# EUROPEAN PATENT SPECIFICATION

(11) **EP 1 368 367 B1**
(45) Date of publication and mention of the grant of the patent: **03.05.2017**
(21) Application number: 02709367.3
(22) Date of filing: 08.02.2002
(51) Int. Cl.: C12N 15/53, C12N 15/54, C12N 15/60, C12N 15/63, C12N 9/02, C12N 9/12, C12N 9/88, C12N 1/20, C12N 5/10, C12P 13/08

(54) **POLYNUCLEOTIDE CONSTRUCTS FOR INCREASED LYSINE PRODUCTION**
POLYNUKLEOTIDKONSTRUKTE FÜR EINE ERHÖHTE LYSINPRODUKTION
CONSTRUCTIONS DE POLYNUCLEOTIDES DESTINEES A UNE PRODUCTION DE LYSINE AMELIOREE

(30) Priority: 08.02.2001 US 267183 P
(43) Date of publication of application: 10.12.2003
(73) Proprietor: ARCHER-DANIELS-MIDLAND COMPANY, Decatur, IL 62525 (US)
(72) Inventor: LI, Lhing-Yew, Savoy, IL 61847 (US); TREI, Kelli, Jean, Decatur, IL 62521 (US)
(74) Representative: Grünecker Patent- und Rechtsanwälte PartG mbB
(86) International application number: PCT/US2002/003469
(87) International publication number: WO 2002/064610

(56) References cited:
- EP-A- 0 387 527
- EP-A- 0 733 710
- EP-A- 0 841 395
- WO-A2-01/49854
- US-A- 6 040 160
- PISABARRO A ET AL: "A CLUSTER OF THREE GENES (DAPA, ORF2, AND DAPB) OF BREVIBACTERIUM LACTOFERMENTUM ENCODES DIHYDRODIPICOLINATE SYNTHASE, DIHYDRODIPICOLINATE REDUCTASE, AND A THIRD POLYPEPTIDE OF UNKNOWN FUNCTION" JOURNAL OF BACTERIOLOGY, WASHINGTON, DC, US, vol. 175, no. 9, 1 May 1993 (1993-05-01), pages 2743-2749, XP002037685 ISSN: 0021-9193
- JETTEN M S M ET AL: "Effect of different levels of aspartokinase on the lysine production by Corynebacterium lactofermentum" APPLIED MICROBIOLOGY AND BIOTECHNOLOGY, SPRINGER VERLAG, BERLIN, DE, vol. 43, no. 1, 1995, pages 76-82, XP008031159 ISSN: 0175-7598
- CREMER ET AL.: 'Control of the lysine biosynthesis sequence in corynebacterium glutamicum as analyzed by overexpression of the individual corresponding genes' vol. 57, no. 6, June 1991, pages 1746 - 1752, XP000616281
- MARCEL ET AL.: 'Nucleotide sequence and organization of the upstream region of the corynebacterium glutamicum lysA gene' MOLECULAR MICROBIOLOGY vol. 4, no. 11, 1990, pages 1819 - 1830, XP000993126
- MALUMBRES ET AL.: 'Molecular control mechanisms of lysine and threonine biosynthesis in amino acid-producing corynebacteria: redirecting carbon flow' vol. 143, 1996, pages 103 - 114, XP001056611

## Description

### BACKGROUND OF THE INVENTION

### Field of the Invention

The invention relates to production of lysine, and provides several isolated polynucleotide molecules useful for the production of L-lysine. One such polynucleotide encodes an aspartate kinase (ask), an aspartate-semialdehyde dehydrogenase (asd) and a dihydrodipicolinate reductase (dapB). Other polynucleotides encode ask, asd, dapB and a diaminopimelate dehydrogenase (ddh); ask, asd, dapB, ddh and an ORF2 polypeptide; and ask, asd dapB, ddh, ORF2 and a diaminopimelate decarboxylase (lysA). The invention further provides isolated host cells and vectors useful for the production of L-Lysine. Disclosed herein are methods of making and using the polynucleotides, and methods to increase the production of L-lysine.

### Related Art

L-lysine is an important economic product obtained principally by industrial-scale fermentation utilizing the Gram positive *Corynebacterium glutamicum*, *Brevibacterium flavum* and *Brevibacterium lactofermentum* (Kleemann, A., et. al., Amino Acids, in ULLMANN'S ENCYCLOPEDIA OF INDUSTRIAL CHEMISTRY, vol. A2, pp.57-97, Weinham: VCH-Verlagsgesellschaft (1985)).

The stereospecificity of the amino acids produced by fermentation makes the process advantageous compared with synthetic processes; generally L-form amino acids are produced by the microbial fermentation process. The production of L-lysine and other amino acids through fermentation, utilizing cheap carbon sources such as molasses, glucose, acetic acid and ethanol, is a relatively inexpensive means of production.

Several fermentation processes utilizing various strains isolated for auxotrophic or resistance properties are known in the art for the production of L-lysine: U.S. Patent No. 2,979,439 discloses mutants requiring amino acid supplementation (homoserine, or L-methionine and L- threonine); U.S. Patent No. 3,700,557 discloses mutants having a nutritional requirement for L-threonine, L-methionine, L-arginine, L-histidine, L-leucine, L-isoleucine, L-phenylalanine, L-cystine, or L-cysteine; U.S. Patent No. 3,707,441 discloses a mutant having a resistance to an L-lysine analog; U.S. Patent No. 3,687,810 discloses a mutant having both an ability to produce L-lysine and a resistance to bacitracin, penicillin G or polymyxin; U.S. Patent No. 3,708,395 discloses mutants having a nutritional requirement for homoserine, L-threonine, L-threonine and L-methionine, L-leucine, L-isoleucine or mixtures thereof and a resistance to L-lysine, L-threonine, L-isoleucine or analogs thereof; U.S. Patent No. 3,825,472 discloses a mutant having a resistance to an L-lysine analog; U.S. Patent No. 4,169,763 discloses mutant strains of *Corynebacterium* that produce L-lysine and are resistant to at least one ofaspartic analogs and sulfa drugs; U.S. Patent No. 5,846,790 discloses a mutant strain able to produce L-glutamic acid and L-lysine in the absence of any biotin action-suppressing agent; and U.S. Patent No. 5,650,304 discloses a strain belonging to the genus *Corynebacterium* or *Brevibacterium* for the production of L-lysine that is resistant to 4-N-(D-alanyl)-2,4-diamino-2,4-dideoxy-L-arabinose 2,4-dideoxy-L-arabinose or a derivative thereof.

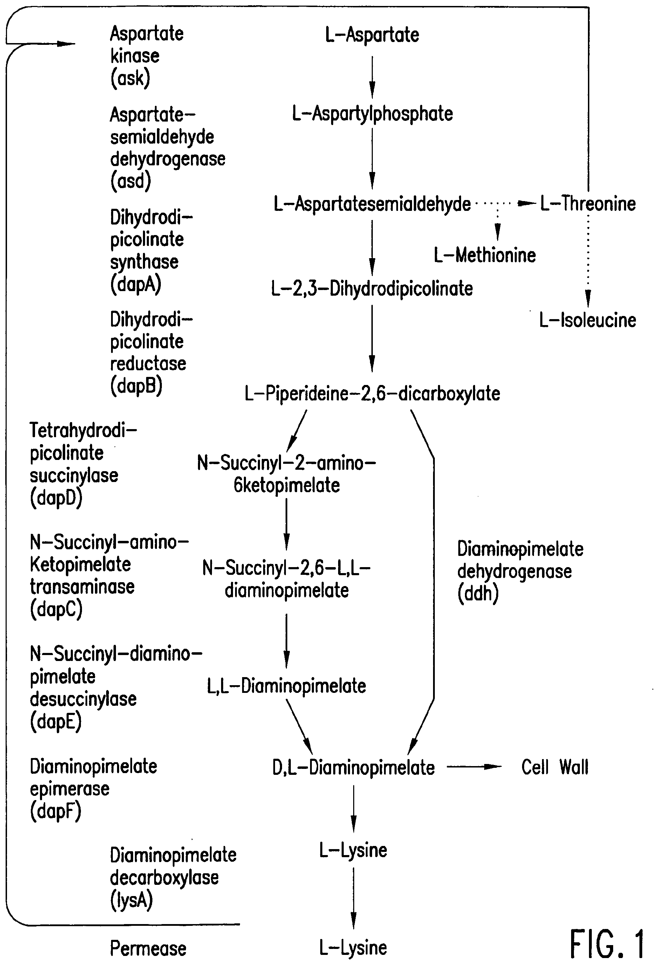

A considerable amount is known regarding the biochemical pathway for L-lysine synthesis in *Corynebacterium* species (recently reviewed by Sahm et al., Ann. N. Y. Acad. Sci. 782: 25-39 (1996)). Entry into the L-lysine pathway begins with L-aspartate (see Figure 1), which itself is produced by transamination of oxaloacetate. A special feature of *C*. *glutamicum* is its ability to convert the L-lysine intermediate piperidine 2,6-dicarboxylate to diaminopimelate by two different routes, i.e. by reactions involving succinylated intermediates or by the single reaction of diaminopimelate dehydrogenase. Overall, carbon flux into the pathway is regulated at two points: first, through feedback inhibition of aspartate kinase by the levels of both L-threonine and L-lysine; and second through the control of the level of dihydrodipicolinate synthase. Therefore, increased production of L-lysine can be obtained in *Corynebacterium* species by deregulating and increasing the activity of these two enzymes.

More recent developments in the area of L-lysine fermentative production involve the use of molecular biology techniques to augment L-lysine production. The following examples are provided: U. S. Patent Nos. 4,560,654 and 5,236,831 disclose an L-lysine producing mutant strain obtained by transforming a host *Corynebacterium* or *Brevibacterium* species microorganism which is sensitive to S-(2-aminoethyl)-cysteine with a recombinant DNA molecule wherein a DNA fragment conferring both resistance to S-(2-aminoethyl)-cysteine and L-lysine producing ability is inserted into a vector DNA; U. S. Patent No. 5,766,925 discloses a mutant strain produced by integrating a gene coding for aspartokinase, originating from coryneform bacteria, with desensitized feedback inhibition by L-lysine and L-threonine, into chromosomal DNA of a *Corynebacterium* species bacterium harboring leaky type homoserine dehydrogenase or a *Corynebacterium* species deficient in homoserine dehydrogenase gene; increased L-lysine production is obtained by gene amplification by way of a plasmid vector or utilizing a gene replacement strategy. European Patent Applications EP 0 811 682 A2 and EP 0 854 189 A2 both provide for increased production of L-lysine in *Corynebacterium* species by way of gene amplification based on plasmid copy number.

### SUMMARY OF THE INVENTION

It is an object of the invention to provide an isolated polynucleotide molecule, referred herein as the KDB polynucleotide, comprising a nucleic acid molecule encoding an aspartate kinase (ask) polypeptide; a nucleic acid molecule encoding an aspartate-semialdehyde dehydrogenase (asd) polypeptide and a nucleic acid molecule encoding a dihydrodipicolinate reductase (dapB) polypeptide. The polynucleotide may further comprise a nucleic acid encoding a complete or truncated diaminopimelate dehydrogenase (ddh) polypeptide (the KDBH polynucleotide), or a nucleic acid encoding a complete or truncated ORF2 polypeptide (the KDB2 polynucleotide). In addition, the invention provides an isolated polynucleotide molecule, referred herein as the KDB2HL polynucleotide, comprising a nucleic acid molecule encoding an ask, asd, dapB, ddh, ORF2 and diaminopimelate decarboxylase (lysA) polypeptides, in which the ddh, ORF2 and lysA polypeptides may be complete or truncated. In a preferred embodiment, a polynucleotide molecule of the invention further comprises a P1 promoter adjacent to the 5' end of the nucleotide molecule encoding lysA.

The subject matter of the invention is as set out in the appended claims.

Accordingly, one aspect of the invention provides an isolated polynucleotide molecule comprising a first nucleic acid with SEQ ID NO: 1, a second nucleic acid with SEQ ID NO: 3, a third nucleic acid with SEQ ID NO: 5 and a fourth nucleic acid with SEQ ID NO. 7.

A further aspect the invention provides an isolated host cell, wherein the host cell is selected from the group consisting of the cells deposited as NRRL-B30410, NRRL-B30458, NRRL-B30459 and NRRL-B30522.

In yet a further aspect the invention provides a vector selected from the group consisting of pDElia2_{FC5}-KDB, pK184-KDBH, pDElia2_{FC5}-KDB2 and pDElia2_{FC5}-KDB2HP1L.

Disclosed herein is a method of increased L-lysine production in a host cell by transforming a host cell with the polynucleotide molecules described above. According to the method disclosed herein, the isolated polynucleotide molecules described above are stably integrated into a chromosome of the host cell, or are maintained as extrachromosomal DNA, such as a plasmid, and the transformed host cells are selected for increased L-lysine production.

### BRIEF DESCRIPTION OF THE FIGURES

Figure 1. A schematic of the L-lysine biosynthetic pathway in *Corynebucterium glutamicum.*

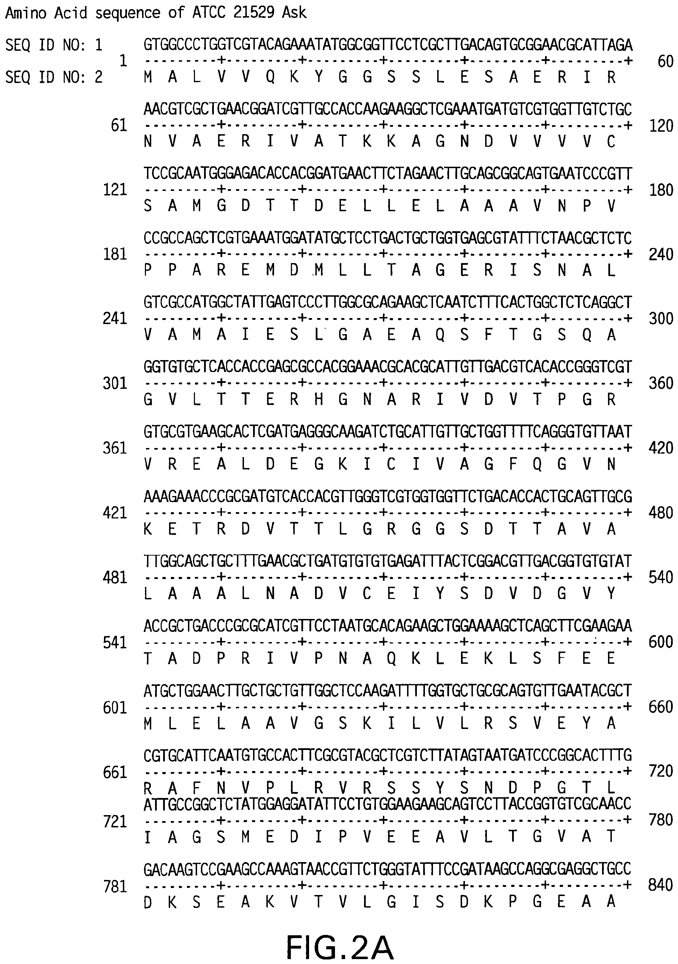

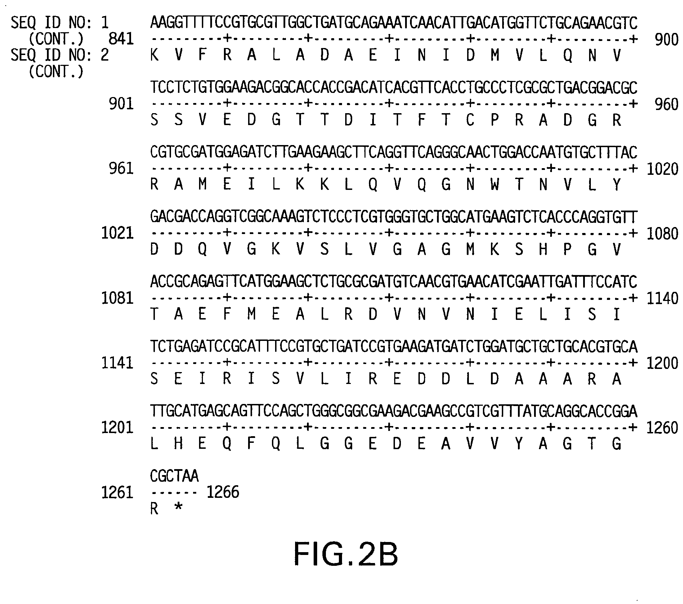

Figure 2 A, B. The nucleotide (SEQ ID NO:1) and amino acid sequence (SEQ ID NO:2) of *ask* (ATCC 21529 sequence).

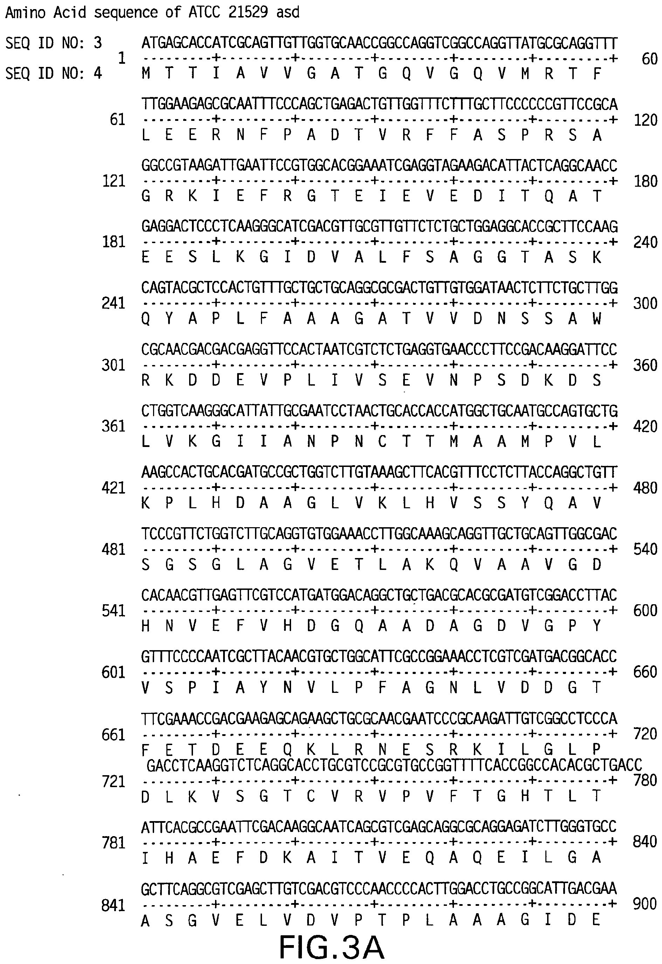

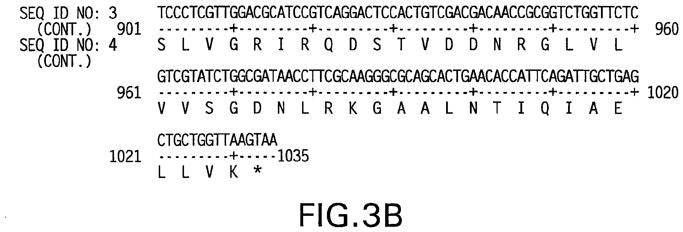

Figure 3 A, B. The nucleotide (SEQ ID NO:3) and amino acid sequence (SEQ ID NO:4) of *asd* (ATCC 21529 sequence).

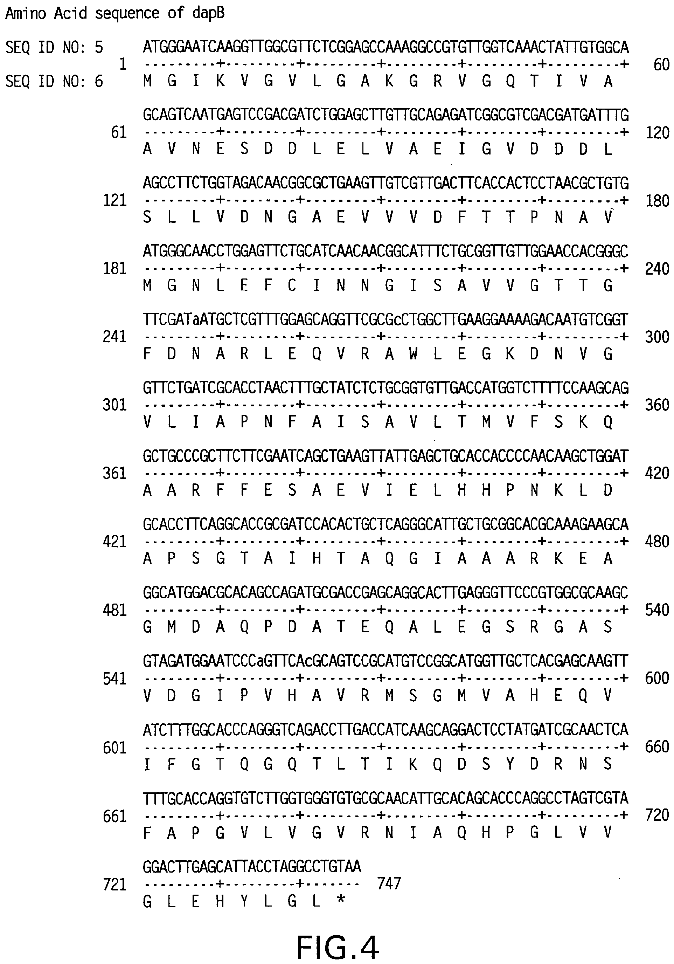

Figure 4. The nucleotide (SEQ ID NO:5) and amino acid sequence (SEQ ID NO:6) of *dapB* (NRRL-B11474).

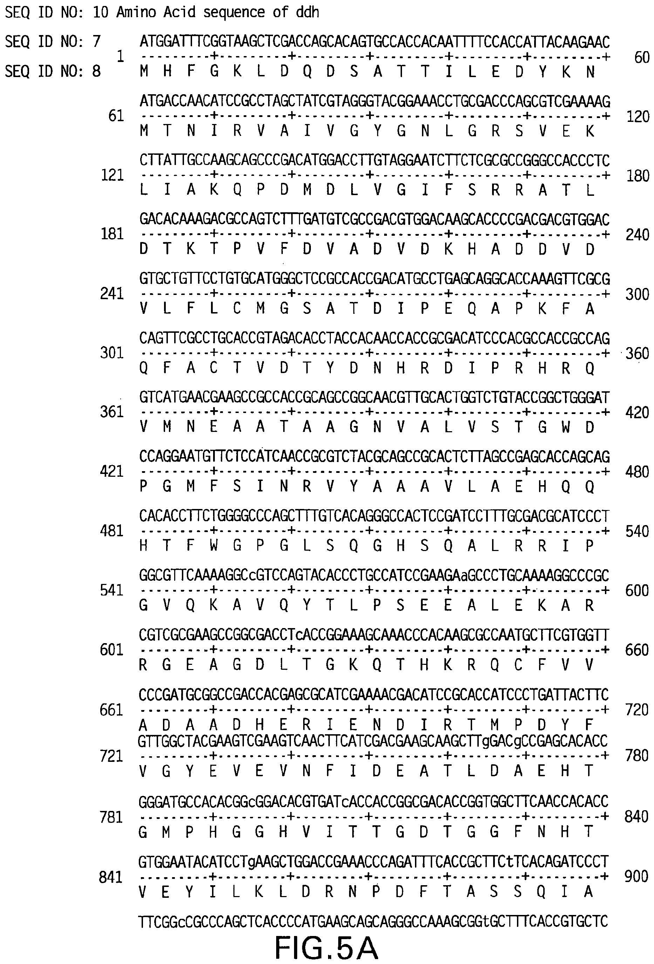

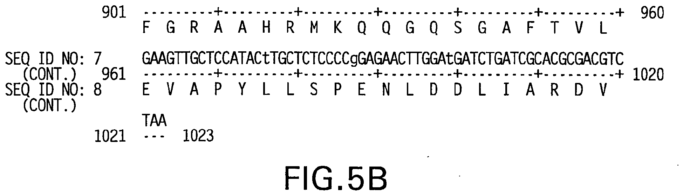

Figure 5 A, B. The nucleotide (SEQ ID NO:7) and amino acid sequence (SEQ ID NO:8) of *ddh* (NRRL-B11474).

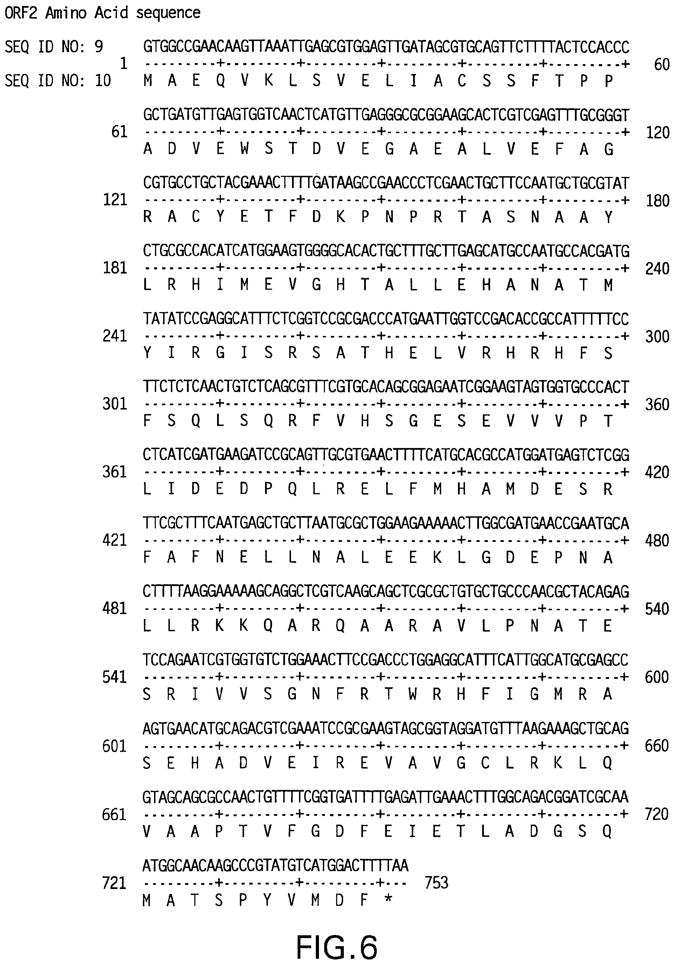

Figure 6. The nucleotide (SEQ ID NO: 9) and amino acid sequence (SEQ ID NO: 10) of ORF2.

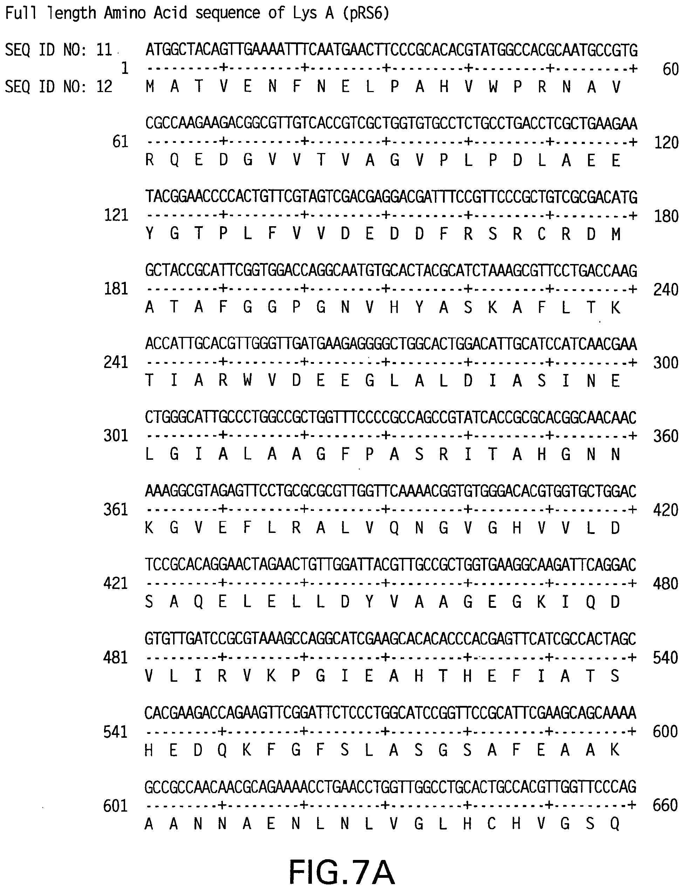

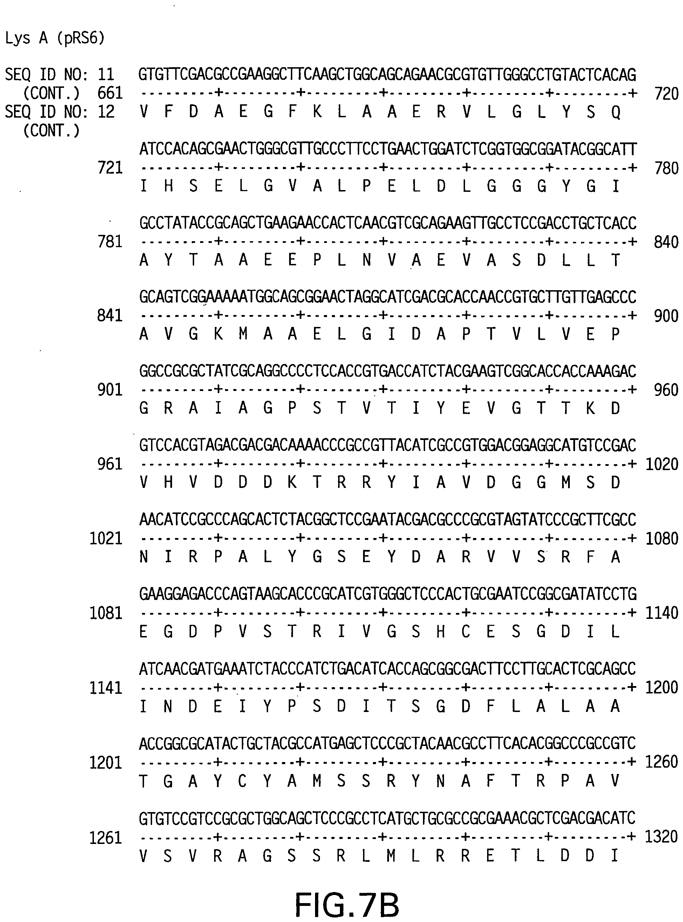

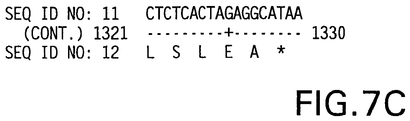

Figure 7 A, B, C. The nucleotide (SEQ ID NO: 11) and amino acid sequence (SEQ ID NO: 12) of lysA.

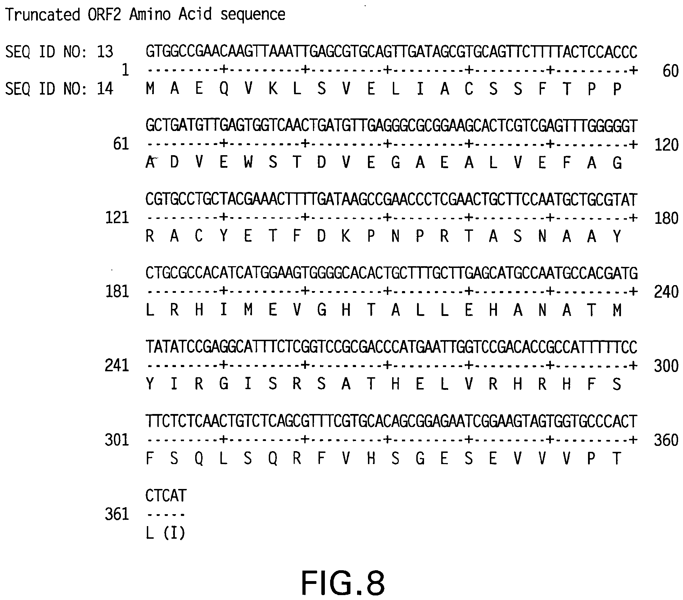

Figure 8. The nucleotide (SEQ ID NO: 13) and amino acid sequence (SEQ ID NO: 14) of truncated ORF2.

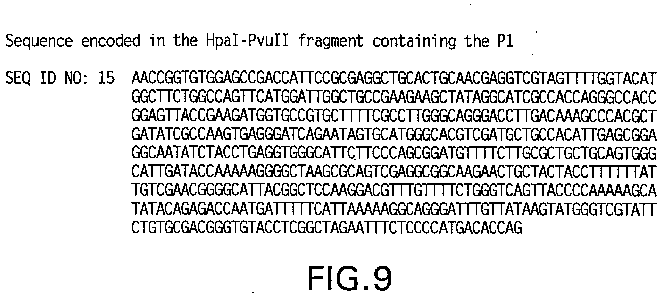

Figure 9. The nucleotide sequence (SEQ ID NO: 15) of the P1 promoter, the first promoter of the argS-lysA operon from pRS6.

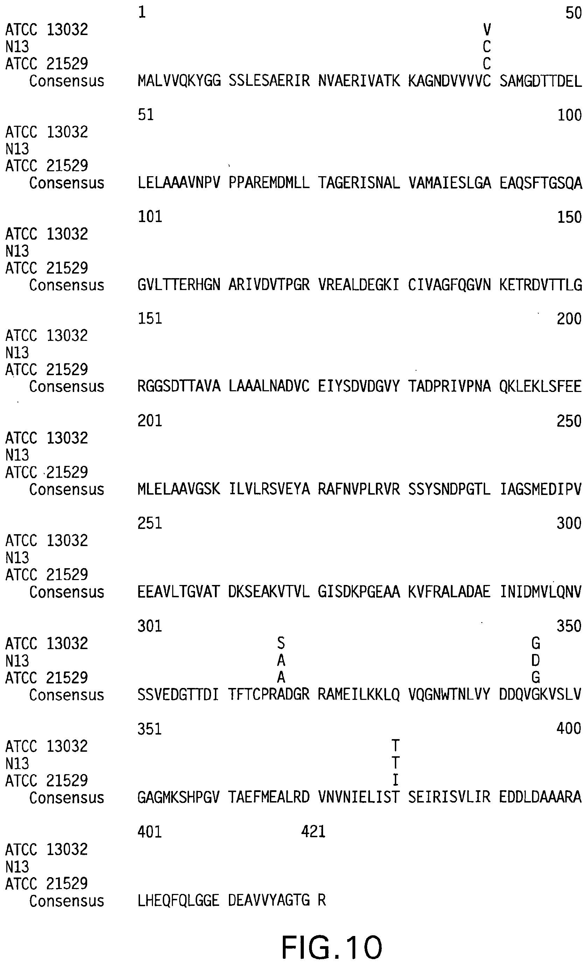

Figure 10. Comparison of the aspartokinase (*ask*) amino acid sequence from ATCC13032, N13 and ATCC21529.
Figure 11A and B. A schematic of the construction of the pDElia2_{FC5}-KDB construct.
Figure 12. A schematic of the construction of the pK184-KDBH construct.
Figure 13. A schematic of the construction of the pDElia2_{FC5}-KDB2 construct.
Figure 14 A, B. A schematic of the construction of the pDElia2_{FC5}-KDB2HP1L construct.

### DETAILED DESCRIPTION OF THE PREFERRED EMBODIMENTS

### A. Definitions

In order to provide a clear and consistent understanding of the specification and claims, including the scope to be given such terms, the following definitions are provided. It is also to be noted that the term "a" or "an" entity, refers to one or more of that entity; for example, "a polynucleotide," is understood to represent one or more polynucleotides.

**Auxotroph.** As used herein, the term refers to a strain of microorganism requiring for growth an external source of a specific metabolite that cannot be synthesized because of an acquired genetic defect.

**Amino Acid Supplement.** As used herein, the term refers to an amino acid required for growth and added to minimal media to support auxotroph growth.

**Chromosomal Integration.** As used herein, the term refers to the insertion of an exogenous DNA fragment into the chromosome of a host organism; more particularly, the term is used to refer to homologous recombination between an exogenous DNA fragment and the appropriate region of the host cell chromosome.

**High Yield Derivative.** As used herein, the term refers to strain of microorganism that produces a higher yield from dextrose of a specific amino acid when compared with the parental strain from which it is derived.

**Host Cell.** As used herein, the term "host cell" is intended to be interchangeable with the term "microorganism." Where a difference is intended, the difference will be made clear.

**Isolated Nucleic Acid Molecule.** As used herein, the term is intended to mean a nucleic acid molecule, DNA or RNA, which has been removed from its native environment. For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically.

**Lysine Biosynthetic Pathway Genes.** As used herein, the term "lysine biosynthetic pathway gene(s)" is meant to include those genes and genes fragments encoding peptides, polypeptides, proteins, and enzymes, which are directly involved in the synthesis of lysine. These genes can be identical to those which naturally occur within a host cell and are involved in the synthesis of lysine within that host cell. Alternatively, there can be modifications or mutations of such genes, for example, the genes can contain modifications or mutations which do not significantly affect the biological activity of the encoded protein. For example, the natural gene can be modified by mutagenesis or by introducing or substituting one or more nucleotides or by removing nonessential regions of the gene. Such modifications are readily performed by standard techniques.

**Lysine Biosynthetic Pathway Protein.** As used herein, the term "lysine biosynthetic pathway protein" is meant to include those peptides, polypeptides, proteins, and enzymes, which are directly involved in the synthesis of lysine from aspartate. Also included are amino acid sequences as encoded by open reading frames (ORF), where the ORF is associated with a lysine biosynthetic pathway operon. These proteins can be identical to those which naturally occur within a host cell and are involved in the synthesis of lysine within that host cell. Alternatively, there can be modifications or mutations of such proteins, for example, the proteins can contain modifications or mutations which do not significantly affect the biological activity of the protein. For example, the natural protein can be modified by mutagenesis or by introducing or substituting one or more amino acids, preferably by conservative amino acid substitution, or by removing nonessential regions of the protein. Such modifications are readily performed by standard techniques. Alternatively, lysine biosynthetic proteins can be heterologous to the particular host cell. Such proteins can be from any organism having genes encoding proteins having the same, or similar, biosynthetic roles.

**Mutagenesis.** As used herein, the term refers to a process whereby a mutation is generated in DNA. With "random" mutagenesis, the exact site of mutation is not predictable, occurring anywhere in the genome of the microorganism, and the mutation is brought about as a result of physical damage caused by agents such as radiation or chemical treatment rDNA mutagenesis is directed to a cloned DNA of interest, and it can be random or site-directed.

**Mutation.** As used herein, the term refers to a one or more base pair change, insertion or deletion, or a combination thereof, in the nucleotide sequence of interest.

**Operably Linked.** As used herein, the term "operably linked" refers to a linkage of polynucleotide elements in a functional relationship. A nucleic acid is "operably linked" when it is placed into a functional relationship with another nucleic acid sequence. For instance, a promoter or enhancer is operably linked to a coding sequence if it affects the transcription of the coding sequence. Operably linked means that the DNA sequences being linked are typically contiguous and, where necessary, join two protein coding regions, contiguous and in reading frame. However, since enhancers generally function when separated from the promoter by several kilobases and intronic sequences can be of variable lengths, some polynucleotide elements can be operably linked but not contiguous.

**Operon.** As used herein, the term refers to a contiguous portion of a transcriptional complex in which two or more open reading frames encoding polypeptides are transcribed as a multi-cistronic messenger RNA, controlled by a cis-acting promoter and other cis-acting sequences necessary for efficient transcription, as well as additional cis acting sequences important for efficient transcription and translation (*e.g*., mRNA stability controlling regions and transcription termination regions). The term generally also refers to a unit of gene expression and regulation, including the structural genes and regulatory elements in DNA.

**Parental Strain.** As used herein, the term refers to a strain of host cell subjected to some form of treatment to yield the host cell of the invention.

**Percent Yield From Dextrose.** As used herein, the term refers to the yield of amino acid from dextrose defined by the formula [(g amino acid produced/ g dextrose consumed)* 100] = % Yield.

**Phenotype.** As used herein, the term refers to observable physical characteristics dependent upon the genetic constitution of a host cell.

**Promoter.** As used herein, the term "promoter" has its art-recognized meaning, denoting a portion of a gene containing DNA sequences that provide for the binding of RNA polymerase and initiation of transcription and thus refers to a DNA sequence capable of controlling the expression of a coding sequence or functional RNA. Promoter sequences are commonly, but not always, found in the 5' non-coding regions of genes. In general, a coding sequence is located 3' to a promoter sequence. Sequence elements within promoters that function in the initiation of transcription are often characterized by consensus nucleotide sequences. The promoter sequence consists of proximal and more distal upstream elements (enhancers). As used herein, the term "endogenous promoter" refers to a promoter sequence which is a naturally occurring promoter sequence in that host microorganism. The term "heterologous promoter" refers to a promoter sequence which is a non-naturally occurring promoter sequence in that host microorganism. The heterologous occurring promoter sequence can be from any prokaryotic or eukaryotic organism. A synthetic promoter is a nucleotide sequence, having promoter activity, and not found naturally occurring in nature.

Promoters can be derived in their entirety from a native gene, or be hybrid promoters. Hybrid promoters are composed of different elements derived from different promoters found in nature, or even comprise synthetic DNA segments. Hybrid promoters can be constitutive, inducible or environmentally responsive.

Useful promoters include constitutive and inducible promoters. Many such promoter sequences are known in the art. See, for example, U.S. Pat. Nos. 4,980,285; 5,631,150; 5,707,828; 5,759,828; 5,888,783; 5,919,670, and, Sambrook, et al., Molecular Cloning: A Laboratory Manual, 2nd Ed., Cold Spring Harbor Press (1989). Other useful promoters include promoters which are neither constitutive nor responsive to a specific (or known) inducer molecule. Such promoters can include those that respond to developmental cues (such as growth phase of the culture), or environmental cues (such as pH, osmoticum, heat, or cell density, for example).

Examples of environmental conditions that can affect transcription by inducible promoters include anaerobic conditions, elevated temperature, or the presence of light. It is understood by those skilled in the art that different promoters can direct the expression of a gene in different cell types, or in response to different environmental conditions. Promoters which cause a gene to be expressed in most cell types at most times are commonly referred to as "constitutive promoters." It is further recognized that since in most cases the exact boundaries of regulatory sequences have not been completely defined, DNA fragments of different lengths can have identical or similar promoter activity.

**Relative Growth.** As used herein, the term refers to a measurement providing an assessment of growth by directly comparing growth of a parental strain with that of a progeny strain over a defined time period and with a defined medium.

### B. Microbiological and Recombinant DNA Methodologies

The present invention relates to a KDB polynucleotide, which comprises a nucleic acid encoding an ask polypeptide, a nucleic acid molecule encoding an asd polypeptide and a nucleic acid molecule encoding a dapB polypeptide, wherein "K" represents a nucleotide sequence encoding the ask polypeptide; "D" represents a nucleotide sequence encoding the asd polypeptide; and "B" represents a nucleotide sequence encoding the dapB polypeptide.

In one embodiment the present invention relates to an isolated KDB polynucleotide molecule comprising:
1. a nucleic acid molecule encoding an aspartate kinase (ask) polypeptide;
2. a nucleic acid molecule encoding an aspartate-semialdehyde dehydrogenase (asd) polypeptide; and
3. a nucleic acid molecule encoding a dihydrodipicolinate reductase (dapB) polypeptide.

In another embodiment, the KDB polynucleotide molecule consists essentially of a nucleic acid molecule encoding an ask polypeptide, a nucleic acid molecule encoding an asd polypeptide and a nucleic acid molecule encoding a dapB polypeptide.

The present invention also relates to a KDBH polynucleotide, which comprises a nucleic acid encoding an ask polypeptide, a nucleic acid molecule encoding an asd polypeptide, a nucleic acid molecule encoding a dapB polypeptide and a nucleic acid molecule encoding a ddh polypeptide, wherein "K" represents a nucleotide sequence encoding the ask polypeptide; "D" represents a nucleotide sequence encoding the asd polypeptide; "B" represents a nucleotide sequence encoding the dapB polypeptide; and "H" represents a nucleotide sequence encoding the ddh polypeptide.

In one embodiment, the KDBH polynucleotide molecule additionally comprises a nucleic acid encoding a complete or truncated ORF2 polypeptide.

The present invention also relates to a KDB2 polynucleotide, which comprises a nucleic acid encoding an ask polypeptide, a nucleic acid molecule encoding an asd polypeptide, a nucleic acid molecule encoding a dapB polypeptide and a nucleic acid molecule encoding an ORF2 polypeptide, and wherein "K" represents a nucleotide sequence encoding the ask polypeptide; "D" represents a nucleotide sequence encoding the asd polypeptide; "B" represents a nucleotide sequence encoding the dapB polypeptide; and "2" represents a nucleotide sequence encoding the ORF2 polypeptide.

The present invention also relates to a KDB2HL polynucleotide, which comprises a nucleotide encoding an ask polypeptide, a nucleic acid molecule encoding an asd polypeptide, a nucleic acid molecule encoding a dapB polypeptide, a nucleic acid molecule encoding an ORF2 polypeptide, a nucleic acid molecule encoding a ddh polypeptide and a nucleic acid molecule encoding a lysA polypeptide. In a preferred embodiment, the KDB2HL polynucleotide molecule also comprises a P1 promoter adjacent to the 5' end of the nucleic acid encoding the lysA polypeptide.

In a preferred embodiment, the polynucleotide molecules of the present invention do not comprise any nucleic acid molecules encoding any lysine pathway polypeptides other than ask, asd, dapB, ddh, ORF2 and lysA.

In one disclosure, an ask polypeptide is defined as a polypeptide having the enzymatic activity of bacterial aspartate kinase. Bacterial aspartate kinase enzymatic activity converts L-aspartate to L-aspartylphosphate. In a further disclosure, an ask polypeptide would have the enzymatic activity of aspartate kinase from ATCC21529. In a further disclosure, the isolated ask amino sequence disclosed in SEQ ID NO:2 possesses unique properties with respect to feedback resistance of ask enzyme activity to accumulated levels of L-lysine and L-threonine in the culture medium. When compared to the DNA sequences of other *Corynebacterium glutamicum ask-asd* gene sequences, a threonine to isoleucine change at amino acid residue 380 which results in resistance to feedback inhibition is observed. Other amino acid changes at residue 380 can also result in decreased ask enzyme sensitivity to L-threonine and/or L-lysine.

An asd polypeptide is defined as a polypeptide having the enzymatic activity of aspartate-semialdehyde dehydrogenase. Aspartate-semialdehyde dehydrogenase enzymatic activity converts L-aspartylphosphate to L-aspartatesemialdehyde. In one disclosure, an asd polypeptide would have the enzymatic activity of aspartate-semialdehyde dehydrogenase from ATCC21529.

A dapB polypeptide is defined as a polypeptide having the enzymatic activity of dihydrodipicolinate reductase. Dihydrodipicolinate reductase enzymatic activity converts L-2,3-dihydrodipicolinate to L-piperideine-2,6-dicarboxylate. In one disclosure, a dapB polypeptide would have the enzymatic activity of dihydrodipicolinate reductase from NRRL-B11474.

A ddh polypeptide is defined as a polypeptide having the enzymatic activity of diaminopimelate dehydrogenase. Diaminopimelate dehydrogenase enzymatic activity converts L-piperideine-2,6-dicarboxylate to D,L-diaminopimelate. In one disclosure, a ddh polypeptide would have the enzymatic activity of diaminopimelate dehydrogenase from NRRL-B11474.

A lysA polypeptide is defined as a polypeptide having the enzymatic activity of diaminopimelate decarboxylase. Diaminopimelate decarboxylase activity converts D,L-diaminopimelate to L-lysine. In one disclosure, a lysA polypeptide would have the enzymatic activity of diaminopimelate decarboxylase from ASO19.

Ask, asd, dapB, ddh, ORF2 and lysA polypeptides encoded by the polynucleotide molecules of the present invention can be truncated forms of the polypeptides encoded by the genomic copies of the ATCC21529 *ask* and *asd genes*, the NRRL-B 11474 *dapB*, *ddh, ORF2* genes and the ASO19 *lysA* gene.

It should be noted that in addition to the indicated polypeptide sequences encoded by the isolated nucleic acid sequences represented by "K", "D", "B," "H," "2" and "L," these isolated nucleic acid sequences can also include native promoter elements for the operons represented therein. Thus, the *ask-asd* sequences can include the respective native *ask-asd* operon elements, and the *dapB* and *ddh* sequences can include their respective native promoter elements. The preferred promoter for the nucleotide molecule encoding the lysA polypeptide is the P1 promoter, the first promoter from the *argS-lysA* operon.

The invention as provided herein utilizes some methods and techniques that are known to those skilled in the arts of microbiology and recombinant DNA technologies. Methods and techniques for the growth of bacterial cells, the introduction of isolated DNA molecules into host cells, and the isolation, cloning and sequencing of isolated nucleic acid molecules, etc., are a few examples of such methods and techniques. These methods and techniques are described in many standard laboratory manuals, such as Davis et al., Basic Methods In Molecular Biology (1986), J.H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, California(1991); J. Sambrook, E.F. Fritsch and T. Maniatis, Molecular Cloning: A Laboratory Manual, 2d ed., Cold Spring Harbor Laboratory Press, Cold Spring Harbor, New York (1989); P.B. Kaufman et al., Handbook of Molecular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Further disclosed is a nucleic acid molecule encoding an ask polypeptide would be at least 90%, 91%, 92%, 93%, 94%, 95%, 96%, 97%, 98% or 99% identical to SEQ ID NO:1. A nucleic acid molecule encoding an asd polypeptide would be at least 90%, 91%, 92%, 93%, 94%, 95%, 96%, 97%, 98% or 99% identical to SEQ ID NO:3. a nucleic acid molecule encoding a dapB polypeptide would be at least 90%, 91%, 92%, 93%, 94%, 95%, 96%, 97%, 98% or 99% identical to SEQ ID NO:5, a nucleic acid molecule encoding a ddh polypeptide would be at least 90%, 91%, 92%, 93%, 94%, 95%, 96%, 97%, 98% or 99% identical to SEQ ID NO:7, a nucleic acid encoding an ORF2 polypeptide would be at least 90%, 91%, 92%, 93%, 94%, 95%, 96%, 97%, 98%, 99% or 100% identical to SEQ ID NO:9 and a nucleic acid encoding a lysA polypeptide would be at least 90%, 91%, 92%, 93%, 94%, 95%, 96%, 97%, 98%, 99% or 100% identical to SEQ ID NO:11 or the complement thereof. In one disclosure the nucleic acid sequence of a P1 promoter would be at least 90%, 91%, 92%, 93%, 94%, 95%, 96%, 97%, 98%, 99% or 100% identical to SEQ ID NO:15. In one disclosure, a nucleic acid encoding a truncated ORF2 polypeptide would be at least 90%, 91%, 92%, 93%, 94%, 95%, 96%, 97%, 98%, 99% or 100% identical to SEQ ID NO:13.

As one skilled in the art would know, any strain of *Corynebacterium* species, particularly that of *Corynebacterium glutamicum,* can be utilized for the isolation of nucleic acid molecules that will be used to amplify the number of chromosomally located amino acid biosynthetic pathway genes. Particularly preferred strains include: NRRL-B11474, ATCC 21799, ATCC 21529, ATCC 21543, and E12.

As a practical matter, whether any particular nucleic acid sequence is at least 90%, 91%, 92%, 93%, 94%, 95%, 96%, 97%, 98% or 99% identical to, for instance, a nucleotide sequence consisting of SEQ ID NO:1, SEQ ID NO:3; SEQ ID NO:5; SEQ ID NO:7; SEQ ID NO:9; SEQ ID NO:11; SEQ ID NO:13; SEQ ID NO:15, or a complementary sequence thereof, can be determined conventionally using sequence analysis computer programs such as a OMIGA® Version 2.0 for Windows, available from Oxford Molecular, Ltd. (Oxford, U.K.). OMIGA uses the CLUSTAL W alignment algorithm using the slow full dynamic programming alignment method with default parameters of an open gap penalty of 10 and an extend gap penalty of 5.0, to find the best alignment between two nucleotide sequences. When using CLUSTAL W or any other sequence alignment program to determine whether a particular sequence is, for instance, 95% identical to a reference sequence according to the present invention, the parameters are set, of course, such that the percentage of identity is calculated over the full length of the reference nucleotide sequence such that gaps, mismatches, or insertions of up to 5% of the total number of nucleotides in the reference sequence are allowed. Other sequence analysis programs, known in the art, can be used in the practice of the invention.

Unless otherwise indicated, all nucleotide sequences described herein were determined using an automated DNA sequencer (such as the Model 373 from Applied Biosystems, Inc.), and all amino acid sequences of polypeptides encoded by DNA molecules described herein were predicted by translation of the relative DNA sequence. Therefore, as is known in the art, for any DNA sequence determined by this automated approach, any nucleotide sequence determined herein can contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art.

It is known in the art that amino acids are encoded at the nucleic acid level by one or more codons (code degeneracy). It is also known in the art that choice of codons may influence expression of a particular amino acid sequence (protein, polypeptide, etc.). Thus, the invention is further directed to nucleic acid molecules encoding the ask amino acid sequence of SEQ ID NO:2 wherein the nucleic acid molecule comprises any codon known to encode a particular amino acid. Likewise, the invention is directed to KDB, KDBH, KDB2 and KDB2HL polynucleotides comprising nucleic acid sequences which comprise alternative codons in order to optimize expression of the protein or polypeptide.

It will be recognized in the art that some disclosed amino acid sequences can be varied without significant effect of the structure or function of the proteins disclosed herein. Variants included can constitute deletions, insertions, inversions, repeats, and type substitutions so long as enzyme activity is not significantly affected. Guidance concerning which amino acid changes are likely to be phenotypically silent can be found in Bowie, J.U., et al., "Deciphering the Message in Protein Sequences: Tolerance to Amino Acid Substitutions," Science 247:1306-1310 (1990),

It is preferred that the polypeptides obtained by the expression of the polynucleotide molecules of the present invention would have at least approximately 80%, 90%, 95%, 96%, 97%, 98%, 99% or 100% identity to one or more amino acid sequences selected from the group comprising SEQ ID No: 2, 4, 6, 8, 10, 12 and 14. A truncated ORF2 polypeptide has at least about 25% of the full length of an ORF2 polypeptide, preferably the ORF2 polypeptide of SEQ ID NO: 10. A truncated ORF2 polypeptide has the sequence of SEQ ID NO: 14. By a polypeptide having an amino acid sequence at least, for example, 95% "identical" to a reference amino acid sequence of a polypeptide is intended that the amino acid sequence of the claimed polypeptide is identical to the reference sequence except that the claimed polypeptide sequence can include up to five amino acid alterations per each 100 amino acids of the reference amino acid of the polypeptide. In other words, to obtain a polypeptide having an amino acid sequence at least 95% identical to a reference amino acid sequence, up to 5% of the amino acid residues in the reference sequence can be deleted or substituted with another amino acid, or a number of amino acids up to 5% of the total amino acid residues in the reference sequence can be inserted into the reference sequence. These alterations of the reference sequence can occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 92%, 95%, 96%, 97%, 98% or 99% identical to, for instance, the amino acid sequence shown in SEQ ID NO:2, 4, 6, 8, 10, 12, 14 or to the amino acid sequence encoded by a nucleic acid sequence can be determined conventionally using known computer programs such the Bestfit program (Wisconsin Sequence Analysis Package, Version 8 for Unix, Genetics Computer Group, University Research Park, 575 Science Drive, Madison, WI 53711). When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

The identity between a reference sequence (query sequence, a sequence of the present invention) and a subject sequence, also referred to as a global sequence alignment, is determined using the FASTDB computer program based on the algorithm of Brutlag et al. (Comp. App. Biosci. 6:237-245 (1990)). Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. Accordingly, if the subject sequence is shorter than the query sequence due to N- or C-terminal deletions, not because of internal deletions, a manual correction is made to the results to take into consideration the fact that the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. A determination of whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of this embodiment. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for.

### C. Disclosed Methods and Processes

Disclosed herein are methods of utilizing the KDB, KDBH, KDB2 and KDB2HL polynucleotide molecules. Any one of these polynucleotide molecules is utilized to increase the production of lysine from a host cell.

The amino acid pathway for L-lysine biosynthesis is well known to skilled artisans of amino acid production. Genes encoding the enzymes important for the conversion of L-aspartate to L-lysine include the *ask*, *asd*, *dapA*, *dapB*, *ddh* and *lysA* genes(Figure 1). Thus, the application utilizes L-lysine biosynthetic pathway genes.

The isolated polynucleotide molecules of the invention are preferably propagated and maintained in an appropriate nucleic acid vector. Methods for the isolation and cloning of the isolated nucleic acid molecules of the invention are well known to those skilled in the art of recombinant DNA technology. Appropriate vectors and methods for use with prokaryotic and eukaryotic hosts are described by Sambrook et al., Molecular Cloning: A Laboratory Manual, Second Edition, Cold Spring Harbor, N.Y., 1989.

A great variety of vectors can be used in the invention. Such vectors include chromosomal, episomal and virus-derived vectors, e.g., vectors derived from bacterial plasmids and from bacteriophage, as well as vectors derived from combinations thereof, such as those derived from plasmid and bacteriophage genetic elements, such as cosmids and phagemids, all can be used in accordance with this aspect of the present invention. Retroviral vectors can be replication competent or replication defective. In the latter case, viral propagation generally will occur only in complementing host cells. Preferred, are vectors suitable to maintain and propagate a polynucleotide in a bacterial host.

A large numbers of suitable vectors and promoters for use in bacteria are known, many of which are commercially available. Preferred prokaryotic vectors include plasmids such as those capable of replication in *E. coli* (such as, for example, pBR322, ColEl, pSC101, pACYC 184, πVX). Such plasmids are, for example, disclosed by Maniatis, T., et al., In: Molecular Cloning, A Laboratory Manual, Cold Spring Harbor Press, Cold Spring Harbor, NY (1982)). The following vectors are provided by way of example: pET (Novagen), pQE70, pQE60, pQE-9 (Qiagen), pBs, phagescript, psiX174, pBlueScript SK, pBsKS, pNH8a, pNH16a, pNH18a, pNH46a (Stratagene), pTrc99A, pKK223-3, pKK233-3, pDR540, pRIT5 (Pharmacia).

Preferred vectors for the isolated nucleic acid molecules of the invention include the pFC1 to pFC7 novel family of combinatorial cloning vectors (Lonsdale, D.M., et al., Plant Molecular Biology Reporter 13: 343-345 (1995)) and the pK 184 vector (Jobling, M.G. and Homes, R.K., Nucleic Acid Research 18: 5315-5316 (1990)).

Another group of preferred vectors are those that are capable of autonomous replication in *Corynebacterium* species. Such vectors are well known to those skilled in the art of amino acid production by way of microbial fermentation, examples of which include pSR1, pMF1014α and vectors derived therefrom. Other suitable vectors will be readily apparent to the skilled artisan.

A KDB, KDBH, KDB2 or KDB2HL polynucleotide can be joined to a vector containing a selectable marker for propagation in a host. The vectors can include at least one selectable marker. In this regard, vectors preferably contain one or more selectable marker genes to provide a phenotypic trait for selection of transformed host cells. Such markers include dihydrofolate reductase, G418 or neomycin resistance for eukaryotic cell culture and tetracycline, kanamycin or ampicillin resistance genes, or an autotrophic gene which allows the host cell to grow in the absence of a nutrient for which the host cell strain is normally autotrophic.

Representative examples of appropriate hosts include, but are not limited to, bacterial cells, such as *E. coli*, *Streptomyces* and *Salmonella typhimurium* cells; fungal cells, such as yeast cells and insect cells such as Drosophila S2 and Spodoptera Sf9 cells. Appropriate culture mediums and conditions for the above-described host cells are known in the art.

If the vector is intended to be maintained in the host cell extrachromosomally, it will contain, in addition and origin of replication which will allow it to replicate in the host cell. Alternatively, if it is desired that the vector integrate into the chromosome, the vector is constructed such that it cannot replicate in the host cell. For example, such a vector might be capable of propagation in another organism, for example, *E. coli,* but lack the proper origin of replication to be propagated in *Corynebucterium.* In another aspect of this embodiment, the vector is a shuttle vector which can replicate and be maintained in more than one host cell species, for example, such a shuttle vector might be capable of replication in a *Corynebacterium* host cell such as a *C*. *glutamicum* host cell, and also in an *E. coli* host cell.

The additional copies of the L-lysine biosynthesis pathway gene(s) selected from *ask, asd, dapB, ddh, ORF2* and *lysA* can be integrated into the chromosome. Furthermore, the additional copies of the L-lysine biosynthesis pathway gene(s) are carried extra-chromosomally. Amplifications by a factor of 5 or less can be obtained by introducing the additional gene copies into the chromosome of the host strain by way of single event homologous recombination. The recombination event results in the introduction of one additional copy of the copy of the gene or genes of interest. If more than 5 copies of the genes are desired, multicopy plasmids carrying the recombinant DNA construct of the invention can be utilized.

Enzyme activity is increased by overexpressing one or more genes of the group comprising *ask, asd, dapB, ddh, ORF2* and *lysA* encoding one or more lysine biosynthetic pathway enzymes. In one embodiment of the invention, said one or more genes are operably linked directly or indirectly to one or more promoter sequences. In another embodiment of the invention, said operably linked promoter sequences are heterologous, endogenous, or hybrid. In a preferred embodiment of the invention, said promoter sequences are one or more of: a promoter sequence from the 5' end of genes endogenous to *C*. *glutamicum,* a promoter sequence from plasmids that replicate in *C*. *glutamicum,* and, a promoter sequence from the genome of phage which infect C. *glutamicum.* In another embodiment, one or more of said promoter sequences are modified. In another preferred embodiment, said modification comprises truncation at the 5' end, truncation at the 3' end, non-terminal insertion of one or more nucleotides, non-terminal deletion of one or more nucleotides, addition of one or more nucleotides at the 5' end, addition of one or more nucleotides at the 3' end, and, combinations thereof. In a preferred embodiment, the P1 promoter, the first promoter of the *argS-lysA* operon is used as the promoter for the *lysA* gene.

Alternative gene promoter elements can be utilized in the constructs of the invention. For example, known bacterial promoters suitable for this use in the present invention include the *E. coli lacI* and *lacZ* promoters, the *T3* and *T7* promoters, the *gpt* promoter, the lambda *PR* and *PL* promoters, the *trp* promoter, or promoters endogenous to the bacterial cells of the present invention. Other promoters useful in the invention include regulated promoters, unregulated promoters and heterologous promoters. Many such promoters are known to one of skill in the art. See Sambrook, E.F. el al., Molecular Cloning: A Laboratory Manual, 2d ed., Cold Spring Harbor Laboratory Press, Cold Spring Harbor, New York (1989).

In addition, the vector can contain control regions that regulate as well as engender expression. Generally, such regions will operate by controlling transcription, such as inducer or repressor binding sites and enhancers, among others.

In one aspect of the invention, the KDB polynucleotide molecule is encompassed in vector pDElia2_{FC5}-KDB. In another aspect, the KDBH polynucleotide molecule is encompassed in vector pK184-KDBH. In another aspect, the KDB2 polynucleotide molecule is encompassed in vector pDElia2_{FC5}-KDB2. In a further aspect , the KDB2HP1L polynucleotide is encompassed in vector pDElia2_{FC5}-KDB2HP1L.

It is a further object of the invention to provide a host cell comprising a vector comprising any one of the isolated KDB, KDBH, KDB2 or KDB2HP1L polynucleotide molecule.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). If the vector is a virus, it can be packaged in vitro using an appropriate packaging cell line and then transduced into host cells.

Representative examples of appropriate hosts for the above described isolated nucleic acid molecules include, but are not limited to, bacterial cells, such as *C*. *glutamicum, Escherichia coli, Streptomyces* and *Salmonella typhimurium* cells; and fungal cells, such as yeast cells. Appropriate culture media and conditions for the above-described host cells are known in the art.

Bacterial cells, such as *E. coli* and coryneform bacteria are preferred as host cells. Particularly preferred *Corynebacterium* and *Brevibacterium* species of the methods and processes disclosed herein include: *Corynebacterium glutamicum, Brevibacterium flavum, Brevibacterium lactofermentum* and other *Cornynebacteria* and *Brevibacteria* species known in the art.

As will be understood by those skilled in the art, the term "*Corynebacterium* species" includes those organisms previously identified in the literature as *"Brevibacterium* species," for example *Brevibacterium flavum* and *Brevibacterium lactofermentum* which have now been reclassified into the genus *Corynebacterium* (Int. J. Syst. Bacteriol. 41: 255 (1981)).

The invention further provides a host cell wherein said host cell is a *Brevibacterium* selected from the group consisting of *Brevibacterium flavum* NRRL-B30458, *Brevibacterium flavum* NRRL-B30410, *Brevibacterium flavum* NRRL-B30459, and *Brevibacterium flavum* NRRL-B30522. Further disclosed are *Brevibacterium flavum* NRRL-B30218, *Brevibacterium flavum* NRRL-B30219, *Brevibacterium lactofermentum* NRRL-B30220, *Brevibacterium lactofermentum* NRRL-B30221, *Brevibacterium lactofermentum* NRRL-B30222, *Brevibacterium flavum* NRRL-B30234 and *Brevibacterium lactofermentum* NRRL-B30235. In a further disclosure, the host cell is *Escherichia coli.* In another disclosure, the host cell is *E. coli* DH5 a MCR NRRL-B30228. In another disclosure, the host cell is a *C*. *glutamicum* selected from the group consisting of *C*. *glutamicum* NRRL-B30236 and *C*. *glutamicum* NRRL-B30237.

The methods to increase the production of lysine and the processes for the production of lysine disclosed herein can both utilize a step requiring the transformation of an isolated nucleic acid molecule into a host cell.

The methods to increase the production of lysine and the processes for the production of lysine disclosed herein can utilize a step requiring amplification of at least one lysine biosynthesis pathway gene. As known to one skilled in the art, the term amplification means increasing the number of a gene or genes of lysine biosynthetic pathway by any means known in the art. Particularly preferred means of amplification include: (1) the addition an isolated KDB, KDBH, KDB2 or KDB2HL polynucleic acid molecule by insertion into the chromosome of a host cell, for example by homologous recombination, and (2) the addition an isolated KDB, KDBH, KDB2 or KDB2HL polynucleic acid molecule into a host cell by way of a self-replicating, extra-chromosomal vector, for example, a plasmid.

Methods of inserting an isolated nucleic acid molecule into the chromosome of a host cell are known to those skilled in the art. For example, insertion of isolated nucleic acid molecules into the chromosome of *Corynebacterium* species can be done utilizing the pK184 plasmid described by Jobling, M. et al., Nucleic Acids Research 18(17): 5315-5316 (submitted 1990). Because these vectors lack a *Corynebacterium* species origin of replication and contain a selectable marker such as kanamycin (*kan*), cells will only be capable of growing under selection if the vector has been inserted into the host cell chromosome by homologous recombination.

The application further discloses methods for increasing lysine production and processes for the production of lysine wherein biosynthetic pathway gene amplification is accomplished through the introduction into a host cell of a self-replicating, extra-chromosomal vector, *e.g*., a plasmid, comprising an isolated KDB, KDBH, KDB2 or KDB2HL polynucleotide molecule. Suitable plasmids include pSR1 and other derivatives of pSR1 (Archer, J. et al., J. Gen. Microbiol. 139: 1753-1759 (1993)).

For a method to increase production of L-lysine, screening for increased production of L-lysine, can be determined by directly comparing the amount of L-lysine produced in culture by a *Corynebacterium* species host strain to that of a *Corynebacterium* species transformed host strain in which lysine biosynthesis gene or genes are amplified. The level of production of lysine can conveniently be determined by the following formula to calculate the percent yield from dextrose: [(g lysine/L / (g dextrose consumed/L)] * 100.

The application further discloses a method to increase the production of lysine comprising: (a) transforming a host cell with an isolated KDBH polynucleotide molecule (b) selecting a transformed host cell; and (c) screening for increased production of lysine from said transformed host cell relative to said host cell. The method further comprises growing said transformed host cell in a medium; and purifying lysine produced by said transformed host cell.

A variety of media known to those skilled in the art can be used to support cell growth for the production of lysine. Illustrative examples of suitable carbon sources include, but are not limited to: carbohydrates, such as glucose, fructose, sucrose, starch hydrolysate, cellulose hydrolysate and molasses; organic acids, such as acetic acid, propionic acid, formic acid, malic acid, citric acid, and fumaric acid; and alcohols, such as glycerol. Illustrative examples of suitable nitrogen sources include, but are not limited to: ammonia, including ammonia gas and aqueous ammonia; ammonium salts of inorganic or organic acids, such as ammonium chloride, ammonium phosphate, ammonium sulfate and ammonium acetate; and other nitrogen-containing sources, including meat extract, peptone, corn steep liquor, casein hydrolysate, soybean cake hydrolysate, urea and yeast extract.

A variety of fermentation techniques are known in the art which can be employed in the disclosed processes drawn to the production of amino acids. Generally, amino acids can be commercially produced from the invention in fermentation processes such as the batch type or of the fed-batch type. In batch type fermentations, all nutrients are added at the beginning of the fermentation. In fed-batch or extended fed-batch type fermentations one or a number of nutrients are continuously supplied to the culture, right from the beginning of the fermentation or after the culture has reached a certain age, or when the nutrient(s) which are fed were exhausted from the culture fluid. A variant of the extended batch of fed-batch type fermentation is the repeated fed-batch or fill-and-draw fermentation, where part of the contents of the fermenter is removed at some time, for instance when the fermenter is full, while feeding of a nutrient is continued. In this way a fermentation can be extended for a longer time.

Another type of fermentation, the continuous fermentation or chemostat culture, uses continuous feeding of a complete medium, while culture fluid is continuously orsemi-continuously withdrawn in such a way that the volume of the broth in the fermenter remains approximately constant. A continuous fermentation can in principle be maintained for an infinite time.

In a batch fermentation an organism grows until one of the essential nutrients in the medium becomes exhausted, or until fermentation conditions become unfavorable (*e.g*., the pH decreases to a value inhibitory for microbial growth). In fed-batch fermentations measures are normally taken to maintain favorable growth conditions, *e.g*., by using pH control, and exhaustion of one or more essential nutrients is prevented by feeding these nutrient(s) to the culture. The microorganism will continue to grow, at a growth rate dictated by the rate of nutrient feed. Generally a single nutrient, very often the carbon source, will become limiting for growth. The same principle applies for a continuous fermentation, usually one nutrient in the medium feed is limiting, all other nutrients are in excess. The limiting nutrient will be present in the culture fluid at a very low concentration, often unmeasurably low. Different types of nutrient limitation can be employed. Carbon source limitation is most often used. Other examples are limitation by the nitrogen source, limitation by oxygen, limitation by a specific nutrient such as a vitamin or an amino acid (in case the microorganism is auxotrophic for such a compound), limitation by sulphur and limitation by phosphorous.

Lysine can be recovered by any method known in the art. Exemplary procedures are provided in the following: Van Walsem, H.J. & Thompson, M.C., J. Biotechnol. 59:127-132 (1997), and U.S. Pat. No. 3,565,951.

The pDElia2_{FC5}-KDB, the pK184-KDBH, the pDElia2_{FC5}-KDB2 and the pDElia2_{FC5}-KDB2HL constructs in NRRL-B11474 host cells were deposited at an acceptable International Depositary Authority in accordance with the Budapest Treaty on the International Recognition of the Deposit of Microorganisms for the Purposes of Patent Procedure. The deposits have been made with the Agricultural Research Service, Culture Collection (NRRL), 1815 North University Street, Peoria, Illinois 61604.

### EXAMPLES

### EXAMPLE 1

### Preparation of L-Lysine Pathway Multi-gene Constructs

Constructs comprising a KDB, a KDBH, a KDB2HL or a KDB2 polynucleotide molecule were made from the following sources:

| ***Gene(s)*** | ***Source*** |
|---|---|
| *ask-asd* | Strain ATCC 21529; |
| *dapB* | Strain NRRL B11474; |
| *ddh* | Strain NRRL B11474; |
| *ORF2* | Strain NRRL B11474; |
| *lysA* | Strain ASO19; |

| ***Promoter*** | |
|---|---|
| *P1* | *argS-lysA* operon from pRS6 |

The polymerase chain reaction (PCR) technique was used to construct the KDB, KDBH, KDB2, and KDBHL constructs. Standard PCR and subcloning procedures were utilized in cloning the coding regions of *ask-asd*, *dapB-ORF2-dapA, ddh.*

The primers utilized for cloning experiments included:

| ***Primer name*** | ***Sequence*** |
|---|---|
| *ask* | 5'-GGGTACCTCGCGAAGTAGCACCTGTCAC-3' |
| *asd* | 5'-GCGGATCCCCCATCGCCCCTCAAAGA-3' |
| *dapB* | 5'-AACGGGCGGTGAAGGGCAACT-3' |
| *ORF2* | 5'-GCTCATAGAGTTCAAGGTTACCTTCTTCCC-3' |
| *ddh1* | 5'-CCATGGTACCAAGTGCGTGGCGAG-3' |
| *ddh2* | 5'-CCATGGTACCACACTGTTTCCTTGC-3' |
| *lysA_{(ATG)}* | 5'-CCGGAGAAGATGTAACAATGGCTAC-3' |
| *lysA3B* | 5'-CCTCGACTGCAGACCCCTAGACACC-3' |
| *dapA* | 5'-TGAAAGACAGGGGTATCCAGA-3' |

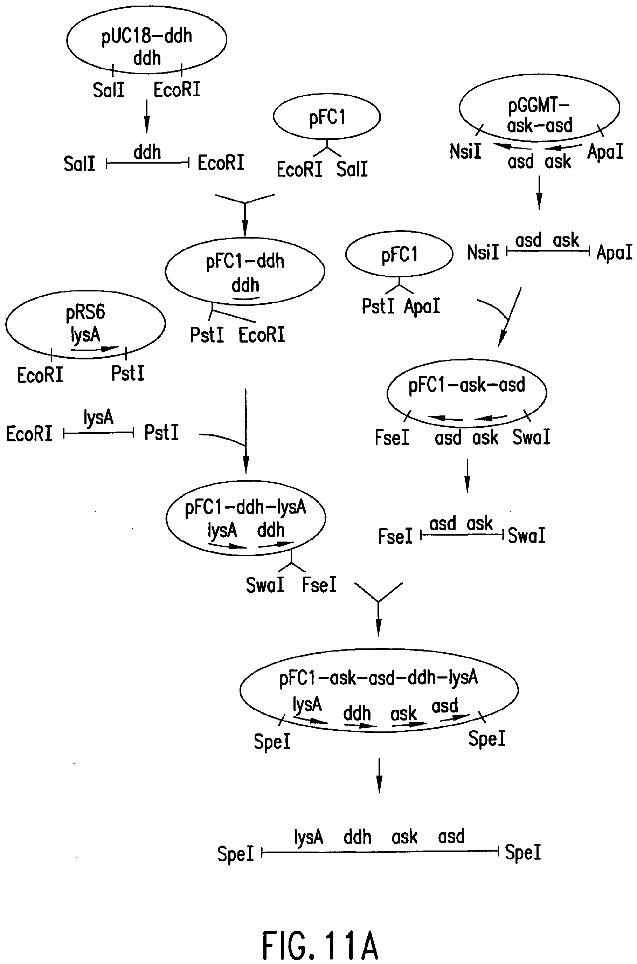

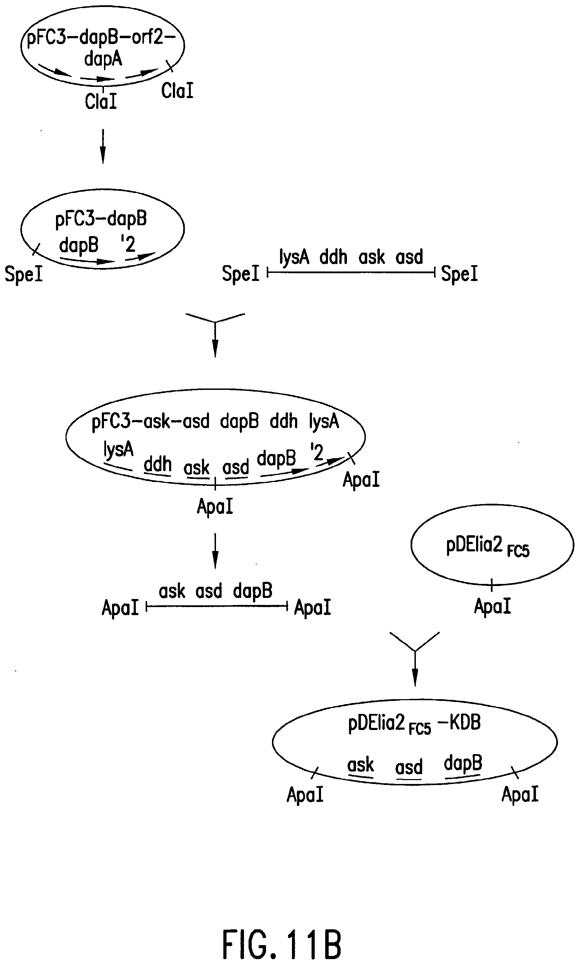

Construction procedures and intermediate plasmids are described in Figures 11-14. The following steps (Figure II) were performed in constructing the pDElia2_{FC5}-KDB vector:
1. pGEMT-ask-asd: an approximately 2.6 Kb PCR product containing the *ask*-*asd* operon of ATCC21529 using primers *ask* and *asd* was cloned into pGEM-T (Promega pGEM-T vector systems).
2. pFC3-ask-asd: an approximately 2.6 Kb NsiI-ApaI fragment of pGEMT-ask-asd was cloned into pFC3 cut with PstI and ApaI.
3. pFC3-dapB-ORF2-dapA: an approximately 2.9 Kb PCR product of NRRL-B11474 *dapB-ORF2-dapA* coding region was cloned into pFC3 at the EcoRV site.
4. pFC3-dapB: the large ClaI fragment of pFC3-dapB-ORF2-dapA was religated.
5. pUC18-ddh: an approximately 1.3 Kb KpnI fragment of pADM21 containing *ddh* (NRRL-B11474 locus) was subcloned into pUC18 at the KpnI site.
6. pFC1-ddh: an approximately 1.3 Kb SalI-EcoRI fragment of pUC18-ddh was cloned into pFC1 cut with SalI and EcoRI.
7. pFC1-ddh-lysA: an approximately 2.1 Kb EcoRI-PstI fragment (containing the intact *lysA* DNA) of pRS6 was cloned into pFC1-ddh cut with EcoRI and PstI.
8. pFC1-ask-asd-ddh-lysA: an approximately 2.6 Kb SwaI-FseI fragment of pFC3-ask-asd was cloned into pFC1-ddh-lysA cut with SwaI and FseI.
9. pFC3-ask-asd-dapB-ddh-lysA: an approximately 6 Kb SpeI fragment of pFC1-ask-asd-ddh-lysA was cloned into pFC3-dapB at the SpeI site.
10. pDElia2_{FC5}-ask-asd-dapB-ddh-lysA (pDElia2_{FC5}-KDBHL): an approximately 7.38 Kb NotI-PmeI fragment of pFC3-ask-asd-dapB-ddh-lysA was cloned into pDElia2_{FC5} cut with NotI and PmeI.
11. pDElia2: an approximately 1.24 Kb blunted PstI fragment of pUC4K was ligated with the approximately 1.75 Kb DraI-SspI fragment of pUC19.
12. pDElia2_{FC5}: the small PvuII fragment of pFC5 was ligated with the large PvuII fragment of pDElia2.
13. pDElia2_{FC5}-ask-asd-dapB(pDElia2_{FC5}-KDB): an approximately 4 Kb ApaI fragment of pDElia2_{FC5}-KDBHL was cloned into pDElia2_{FC5} at the ApaI site.

Corynebacterium (NRRL-B11474) containing the pDElia2_{FC5}-KDB construct was deposited at an acceptable International Depositary Authority in accordance with the Budapest Treaty on the International Recognition of the Deposit of Microorganisms for the Purposes of Patent Procedure. The deposit has been made with the Agricultural Research Service, Culture Collection (NRRL), 1815 North University Street, Peoria, Illinois 61604 on February 1, 2001. The deposit is numbered NRRL-B30458.

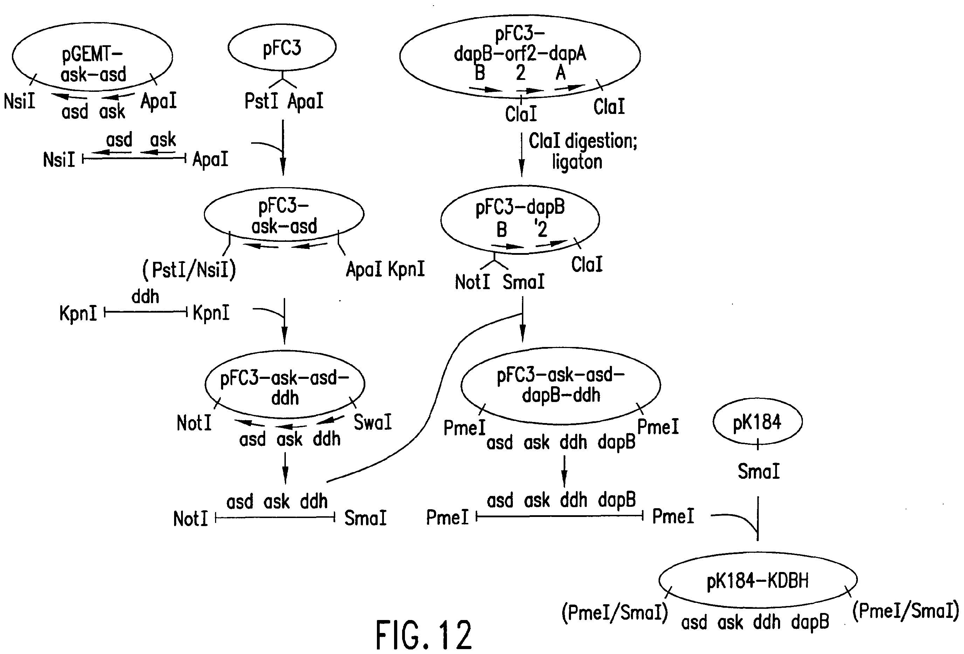

The following steps (Figure 12) were preformed in constructing the pK184-KDBH construct:
1. pGEMT-ask-asd: an approximately 2.6 Kb PCR product containing the *ask-asd* operon of ATCC21529 using primers *ask* and *asd* was cloned into pGEM-T (Promega pGEM-T vector systems).
2. pFC3-ask-asd: an approximately 2.6 Kb Nsil-ApaI fragment of pGEMT-ask-asd was cloned into pFC3 cut with PstI and ApaI.
3. pFC3-ask-asd-ddh: an approximately 1.3 Kb KpnI fragment containing NRRL-B11474 ddh was cloned into pFC3-ask-asd at the KpnI site.
4. pFC3-dapB-ORF2-dapA: an approximately 2.9 Kb PCR product of NRPL-B11474 dapB-ORF2-dapA coding region was cloned into pFC3 at the EcoRV site.
5. pFC3-dapB: the large ClaI fragment of pFC3-dapB-ORF2-dapA was religated.
6. pFC3-ask-asd-dapB-ddh: an approximately 4 Kb NotI-SwaI fragment of pFC3-ask-asd-ddh was cloned into pFC3-dapB digested with NotI and SmaI.
7. pK184-ask-asd-dapB-ddh (pK184-KDBH): an approximately 5.3 Kb PmeI fragment containing ask-asd-dapB-ddh was cloned into pK184 at the SmaI site.

Corynebacterium (NRRL-B11474) containing the pK184-KDBH construct was deposited at an acceptable International Depositary Authority in accordance with the Budapest Treaty on the International Recognition of the Deposit of Microorganisms for the Purposes of Patent Procedure. The deposit has been made with the Agricultural Research Service, Culture Collection (NRRL), 1815 North University Street, Peoria, Illinois 61604 on February 1,2001. The deposit is numbered NRRL-B30410.

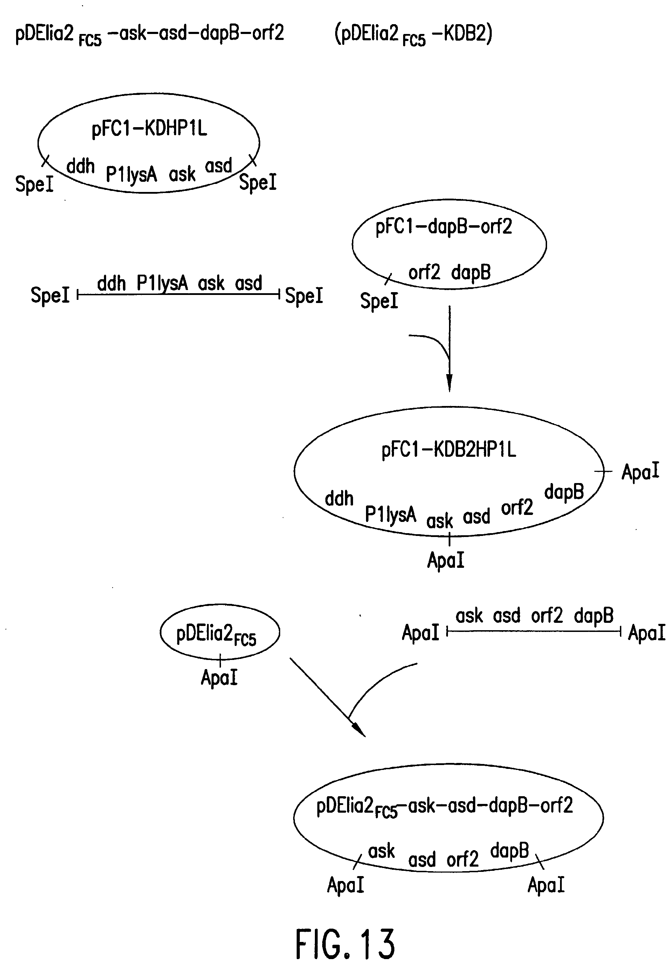

The following steps (Figure 13) were performed in constructing the pDElia2_{FC5}-KDB2 vector:
1. pGEMT-ask-asd: an approximately 2.6 Kb PCR product containing the ask-asd operon of ATCC21529 using primers ask and asd was cloned into pGEM-T (Promega pGEM-T vector systems).
2. pUC18-ddh: an approximately 1.3 Kb KpnI fragment of pADM21 containing ddh (BF100 locus) was subcloned into pUC18 at the KpnI site.
3. pFC3-ask-asd: an approximately 2.6 Kb NsiI-ApaI fragment of pGEMT-ask-asd was cloned into pFC3 cut with PstI and ApaI
4. pFC1-dapB-ORF2: an approximately 2 Kb PCR product of NRRL-B11474 dapB-ORF2 coding region was cloned into pFC1 at the EcoRV site.
5. pFC1-ddh: an approximately 1.3 Kb PstI-EcoRI fragment of pUC18-ddh was cloned into pFC1 cut with PstI and EcoRI.
6. pUC19-P1: an approximately 550 bp HpaI-PvuII fragment (containing the first promoter, P1, of the argS-lysA operon) of pRS6 was cloned into pUC19 at the SmaI site.
7. pUC19-P1lysA: an approximately 1.45 Kb promoterless PCR product, using primers LysA(ATG) and LysA3B, of ASO19 lysA coding region is cloned into pUC19-P1 at the HincII site.
8. pFC1-P1lysA: an approximately 2 Kb EcoRI-HindIII fragment of pUC19-P1lysA was cloned in to pFC1 cut with EcoRI and HindIII.
9. pFC1-ddh-P1lysA: an approximately 1.3 Kb EcoRI-NotI fragment of pFC1-ddh was cloned into pFC1-P1lysA cut with EcoRI and NotI.
10. pFC1-ask-asd-ddh-P1lysA: an approximately 2.6 Kb SwaI-FseI fragment of pFC3-asd-asd was cloned into pFC1-ddh-P1lysA cut with SwaI and Fsel.
10. pFC1-ask-asd-dapB-ORF2-ddh-P1lysA(pFC1-KDB2HPIL): an approximately 5.9 Kb SpeI fragment of pFC1-ask-asd-ddh-P1lysA was cloned into pFC1-dapB-ORF2 at the SpeI site.
11. pDElia2_{FC5}: the small PvuII fragment of pFC5 was ligated with the large PvuII fragment of pDElia2.
12. pDElia2_{FC5}-ask-asd-dapB-ORF2 (pDElia2_{FC5}-KDB2): an approximately 4.7 Kb ApaI fragment containing KDB2 of pFC1-KDB2HP1L was cloned into pDElia2_{FC5} at the ApaI site.

Corynebacterium (NRRL-B11474) containing the pDElia2_{FC5}-KDB2 construct was deposited at an acceptable International Depositary Authority in accordance with the Budapest Treaty on the International Recognition of the Deposit of Microorganisms for the Purposes of Patent Procedure. The deposit has been made with the Agricultural Research Service, Culture Collection (NRRL), 1815 North University Street, Peoria, Illinois 61604 on February 1, 2001. The deposit is numbered NRRL-B30459.

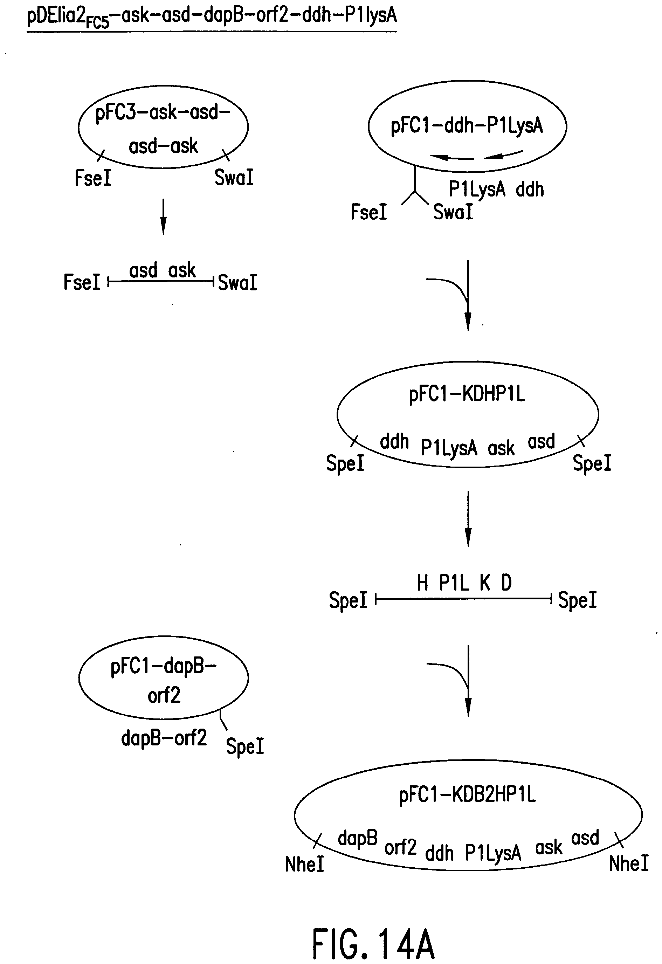

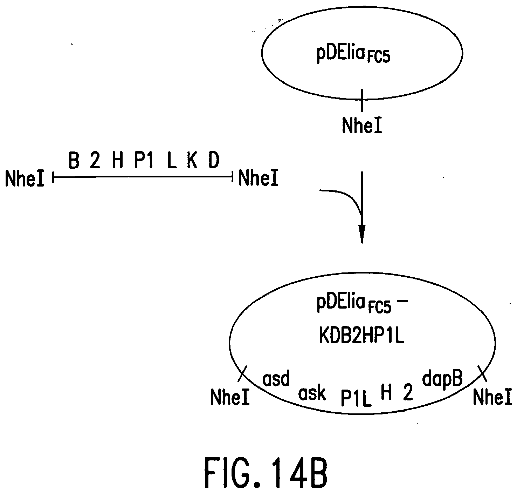

The following steps (Figure 14) were performed in constructing the pDElia2_{FC5}-KDB2HP1L vector:
1. pGEMT-ask-asd: an approximately 2.6 Kb PCR product containing the ask-asd operon of ATCC21529 using primers ask and asd was cloned into pGEM-T (Promega pGEM-T vector systems).
2. pUC 18-ddh: an approximately 1.3 Kb Kpn1 fragment of pADM21 containing ddh (NRRL-B11474 locus) was subcloned into pUC18 at the KpnI site.
3. pFC3-ask-asd: an approximately 2.6 Kb NsiI-Apal fragment of pGEMT-ask-asd was cloned into pFC3 cut with Pstl and Apa1.
4. pFC1-dapB-ORF2: an approximately 2 Kb PCR product of NRRL-B11474 dapB-ORF2 coding region was cloned into pFC1 at the EcoRV site.
5. pFC1-ddh: an approximately 1.3 Kb PstI-EcoRI fragment of pUC19-ddh was cloned into pFC1 cut with PstI and EcoRI.
6. pUC19-P1: an approximately 550 bp HpaI-PvuII fragment (containing the first promoter, P1, of the *argS-lysA* operon) of pRS6 was cloned into pUC19 at the SmaI site.
7. pUC19-P1lysA: an approximately 1.45 Kb promoterless PCR product, using primers LysA(ATG) and LysA3B, of ASO19 lysA coding region is cloned into pUC19-P1 at the HincII site.
8. pFC1-P1lysA: an approximately 2 Kb EcoRI-HindIII fragment of pUC19-P1lysA was cloned into pFC1 cut with EcoRl and HindIII.
9. pFC1-ddh-P1lysA: an approximately 1.3 Kb EcoRI-NotI fragment of pFC1-ddh was cloned into pFC1-P1lysA cut with EcoRI and NotI.
10. pFC1-ask-asd-ddh-P1lysA: an approximately 2.6 Kb SwaI-FseI fragment of pFC3-ask-asd was cloned into pFC1-ddh-P1lysA cut with SwaI and FseI.
11. pFC1-ask-asd-dapB-ORF2-ddh-P1lysA (pFC1-KDB2HP1L): an approximately 5.9 Kb SpeI fragment of pFC1-ask-asd-ddh-P1lysA was cloned into pFC1-dapB-ORF2 at the SpeI site.
12. pDElia2_{FC5}: the small PvuII fragment of pFC5 was ligated with the large PvuII fragment of pDElia2
13. pDElia2_{FC5}-ask-asd-dapB-ORF2-ddh-P1lysA (pDElia2_{FC5}-KDB2HP1L): an approximately 7.9 Kb NHE fragment of pFC1-ask-asd-dapB-ORF2-ddh-P1lysA was cloned into pDElia2_{FC5} at the NHE site.

Corynebacterium (NRRL-B11474) containing the pDElia2_{FC5}-KDB2HP1L construct was deposited at an acceptable International Depositary Authority in accordance with the Budapest Treaty on the International Recognition of the Deposit of Microorganisms for the Purposes of Patent Procedure. The deposit has been made with the Agricultural Research Service, Culture Collection (NRRL), 1815 North University Street, Peoria, Illinois 61604 on February 1, 2001. The deposit is numbered NRRL-B30522.

### EXAMPLE 2

### Screening and Selection of Strains with Improved L-Lysine Production

The production of L-lysine by cells stably transformed with multi-gene constructs is summarized in Table 1.

**Table 1. Lysine production by various parental and stably transfected bacteria**

| **Strain Tested** | **lysine titer (g/L)** | **L-lysine yield (%)** | **Cell Deposit** |
|---|---|---|---|
| NRRL-B11474 | 31 | 30 | |
| NRRL-B11474::pDElia2_{FC5}-KDB | 34 | 37 | NRRL-B30458 |
| | | | |
| NRRL-B11474 | 31 | 31 | |
| NRRL-B11474::pK184-KDBH | 38 | 37.4 | NRRL-B30410 |
| | | | |
| NRRL-B11474 | 30 | 30 | |
| NRRL-B11474::pDElia2_{FC5}-KDB2 | 39 | 37 | NRRL-B30459 |
| | | | |
| NRRL-B11474 | 31 | 33 | |
| NRRL-B11474::pDElia2_{FC5}-KDB2HPIL | 38 | 41 | NRRL-B30522 |

## Claims

1. An isolated polynucleotide molecule comprising:
a) a first nucleic acid with SEQ ID NO: 1;
b) a second nucleic acid with SEQ ID NO: 3;
c) a third nucleic acid with SEQ ID NO: 5; and
d) a fourth nucleic acid with SEQ ID NO: 7.

2. An isolated host cell, wherein said host cell is selected from the group consisting of the cells deposited as NRRL-B30410, NRRL-B30458, NRRL-B30459 and NRRL-B30522.

3. A vector selected from the group consisting of pDElia2_{FC5}-KDB, pK184-KDBH, pDElia2_{FC5}-KDB2, and pDElia2_{FC5}-KDB2HP1L.

## Patentansprüche

1. Ein isoliertes Polynukleotidmolekül umfassend:
(a) eine erste Nukleinsäure mit SEQ ID NO: 1;
(b) eine zweite Nukleinsäure mit SEQ ID NO: 3;
(c) eine dritte Nukleinsäure mit SEQ ID NO: 5; und
(d) eine vierte Nukleinsäure mit SEQ ID NO: 7.

2. Eine isolierte Wirtszelle, wobei besagte Wirtszelle ausgewählt ist aus der Gruppe bestehend aus Zellen hinterlegt als NRRL-B30410, NRRL-B30458, NRRL-B30459 und NRRL-B30522.

3. Ein Vektor ausgewählt aus der Gruppe bestehend aus pDElia2_{FC5}-KDB, pK184-KDBH, pDElia2_{FC5}-KDB2, und pDElia2_{FC5}-KDB2HP1L.

## Revendications

1. Molécule à polynucléotides isolée comprenant :
a) un premier acide nucléique ayant la SEQ ID NO : 1 ;
b) un deuxième acide nucléique ayant la SEQ ID NO : 3 ;
c) un troisième acide nucléique ayant la SEQ ID NO : 5 ; et
d) un quatrième acide nucléique ayant la SEQ ID NO : 7.

2. Cellule hôte isolée, ladite cellule hôte étant choisie dans le groupe constitué des cellules déposées en tant que NRRL-B30410, NRRL-B30458, NRRL-B30459 et NRRL-B30522.

3. Vecteur choisi dans le groupe constitué de pDElia2_{FC5}-KDB, pK184-KDBH, pDElia2_{FC5}-KDB2 et pDElia2_{FC5}-KDB2HP1L.


### SEQUENCE LISTING

<110> Archer-Daniels-Midland Company
<120> Polynucleotide Constructs for Increased Lysine Production
<130> 1533.264PC01
<150> 60/267,183
   <151> 2001-02-08
<160> 25
<170> PatentIn version 3.1
<210> 1
   <211> 1266
   <212> DNA
   <213> Corynebacterium glutamicum
<220>
   <221> CDS
   <222> (1)..(1266)
<400> 1
   gtg gcc ctg gtc gta cag aaa tat ggc ggt tcc tcg ctt gag agt gcg        48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc      240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gct caa tct ttc act      288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc      336
   Gly Ser Gln Ala Gly Val Leu Thr Thr Glu Arg His Gly Asn Ala Arg
               100                 105                 110
   att gtt gac gtc aca ccg ggt cgt gtg cgt gaa gca ctc gat gag ggc      384
   Ile Val Asp Val Thr Pro Gly Arg Val Arg Glu Ala Leu Asp Glu Gly
           115                 120                 125
   aag atc tgc att gtt gct ggt ttt cag ggt gtt aat aaa gaa acc cgc      432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcc aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tcc tct gtg gaa      912
   Ala Glu Ile Asn Ile Asp Met Val Leu Gln Asn Val Ser Ser Val Glu
       290                 295                 300
   gac ggc acc acc gac atc acg ttc acc tgc cct cgc gct gac gga cgc      960
   Asp Gly Thr Thr Asp Ile Thr Phe Thr Cys Pro Arg Ala Asp Gly Arg
   305                 310                 315                 320
   cgt gcg atg gag atc ttg aag aag ctt cag gtt cag ggc aac tgg acc      1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct      1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg      1104
   Gly Met Lys Ser His Pro Gly Val Thr Ala Glu Phe Met Glu Ala Leu
           355                 360                 365
   cgc gat gtc aac gtg aac atc gaa ttg att tcc atc tct gag atc cgc      1152
   Arg Asp Val Asn Val Asn Ile Glu Leu Ile Ser Ile Ser Glu Ile Arg
       370                 375                 380
   att tcc gtg ctg atc cgt gaa gat gat ctg gat gct gct gca cgt gca      1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat      1248
   Leu His Glu Gln Phe Gln Leu Gly Gly Glu Asp Glu Ala Val Val Tyr
                   405                 410                 415
   gca ggc acc gga cgc taa                                              1266
   Ala Gly Thr Gly Arg
               420
<210> 2
   <211> 421
   <212> PRT
   <213> Corynebacterium glutamicum
<400> 2
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
   85 90 95
   Gly Ser Gln Ala Gly Val Leu Thr Thr Glu Arg His Gly Asn Ala Arg
   100 105 110
   Ile Val Asp Val Thr Pro Gly Arg Val Arg Glu Ala Leu Asp Glu Gly
   115 120 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Thr Phe Thr Cys Pro Arg Ala Asp Gly Arg
   305 310 315 320
   Arg Ala Met Glu Ile Leu Lys Lys Leu Gln Val Gln Gly Asn Trp Thr
   325 330 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Ile Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 3
   <211> 1035
   <212> DNA
   <213> Corynebacterium glutamicum
<220>
   <221> CDS
   <222> (1)..(1035)
<400> 3
   atg acc acc atc gca gtt gtt ggt gca acc ggc cag gtc ggc cag gtt       48
   Met Thr Thr Ile Ala Val Val Gly Ala Thr Gly Gln Val Gly Gln Val
   1               5                   10                  15
   atg cgc acc ttt ttg gaa gag cgc aat ttc cca gct gac act gtt cgt       96
   Met Arg Thr Phe Leu Glu Glu Arg Asn Phe Pro Ala Asp Thr Val Arg
               20                  25                  30
   ttc ttt gct tcc ccg cgt tcc gca ggc cgt aag att gaa ttc cgt ggc      144
   Phe Phe Ala Ser Pro Arg Ser Ala Gly Arg Lys Ile Glu Phe Arg Gly
           35                  40                  45
   acg gaa atc gag gta gaa gac att act cag gca acc gag gag tcc ctc      192
   Thr Glu Ile Glu Val Glu Asp Ile Thr Gln Ala Thr Glu Glu Ser Leu
       50                  55                  60
   aag ggc atc gac gtt gcg ttg ttc tct gct gga ggc acc gct tcc aag      240
   Lys Gly Ile Asp Val Ala Leu Phe Ser Ala Gly Gly Thr Ala Ser Lys
   65                  70                  75                  80
   cag tac gct cca ctg ttt gct gct gca ggc gcg act gtt gtg gat aac      288
   Gln Tyr Ala Pro Leu Phe Ala Ala Ala Gly Ala Thr Val Val Asp Asn
                   85                  90                  95
   tct tct gct tgg cgc aag gac gac gag gtt cca cta atc gtc tct gag      336
   Ser Ser Ala Trp Arg Lys Asp Asp Glu Val Pro Leu Ile Val Ser Glu
               100                 105                 110
   gtg aac cct tcc gac aag gat tcc ctg gtc aag ggc att att gcg aat      384
   Val Asn Pro Ser Asp Lys Asp Ser Leu Val Lys Gly Ile Ile Ala Asn
           115                 120                 125
   cct aac tgc acc acc atg gct gca atg cca gtg ctg aag cca ctg cac      432
   Pro Asn Cys Thr Thr Met Ala Ala Met Pro Val Leu Lys Pro Leu His
       130                 135                 140
   gat gcc gct ggt ctt gta aag ctt cac gtt tcc tct tac cag gct gtt      480
   Asp Ala Ala Gly Leu Val Lys Leu His Val Ser Ser Tyr Gln Ala Val
   145                 150                 155                 160
   tcc ggt tct ggt ctt gca ggt gtg gaa acc ttg gca aag cag gtt gct      528
   Ser Gly Ser Gly Leu Ala Gly Val Glu Thr Leu Ala Lys Gln Val Ala
                   165                 170                 175
   gca gtt ggc gac cac aac gtt gag ttc gtc cat gat gga cag gct gct      576
   Ala Val Gly Asp His Asn Val Glu Phe Val His Asp Gly Gln Ala Ala
               180                 185                 190
   gac gca ggc gat gtc gga cct tac gtt tcc cca atc gct tac aac gtg      624
   Asp Ala Gly Asp Val Gly Pro Tyr Val Ser Pro Ile Ala Tyr Asn Val
           195                 200                 205
   ctg cca ttc gcc gga aac ctc gtc gat gac ggc acc ttc gaa acc gac      672
   Leu Pro Phe Ala Gly Asn Leu Val Asp Asp Gly Thr Phe Glu Thr Asp
       210                 215                 220
   gaa gag cag aag ctg cgc aac gaa tcc cgc aag att ctc ggc ctc cca      720
   Glu Glu Gln Lys Leu Arg Asn Glu Ser Arg Lys Ile Leu Gly Leu Pro
   225                 230                 235                 240
   gac ctc aag gtc tca ggc acc tgc gtc cgc gtg ccg gtt ttc acc ggc      768
   Asp Leu Lys Val Ser Gly Thr Cys Val Arg Val Pro Val Phe Thr Gly
                   245                 250                 255
   cac acg ctg acc att cac gcc gaa ttc gac aag gca atc acc gtc gag      816
   His Thr Leu Thr Ile His Ala Glu Phe Asp Lys Ala Ile Thr Val Glu
               260                 265                 270
   cag gcg cag gag atc ttg ggt gcc gct tca ggc gtc gag ctt gtc gac      864
   Gln Ala Gln Glu Ile Leu Gly Ala Ala Ser Gly Val Glu Leu Val Asp
           275                 280                 285
   gtc cca acc cca ctt gca gct gcc ggc att gac gaa tcc ctc gtt gga      912
   Val Pro Thr Pro Leu Ala Ala Ala Gly Ile Asp Glu Ser Leu Val Gly
       290                 295                 300
   cgc atc cgt cag gac tcc act gtc gac gac aac cgc ggt ctg gtt ctc      960
   Arg Ile Arg Gln Asp Ser Thr Val Asp Asp Asn Arg Gly Leu Val Leu
   305                 310                 315                 320
   gtc gta tct ggc gat aac ctt cgc aag ggc gca gca ctg aac acc att     1008
   Val Val Ser Gly Asp Asn Leu Arg Lys Gly Ala Ala Leu Asn Thr Ile
                   325                 330                 335
   cag att gct gag ctg ctg gtt aag taa                                 1035
   Gln Ile Ala Glu Leu Leu Val Lys
               340
<210> 4
   <211> 344
   <212> PRT
   <213> Corynebacterium glutamicum
<400> 4
   Met Thr Thr Ile Ala Val Val Gly Ala Thr Gly Gln Val Gly Gln Val
   1               5                   10                  15
   Met Arg Thr Phe Leu Glu Glu Arg Asn Phe Pro Ala Asp Thr Val Arg
               20                  25                  30
   Phe Phe Ala Ser Pro Arg Ser Ala Gly Arg Lys Ile Glu Phe Arg Gly
           35                  40                  45
   Thr Glu Ile Glu Val Glu Asp Ile Thr Gln Ala Thr Glu Glu Ser Leu
       50                  55                  60
   Lys Gly Ile Asp Val Ala Leu Phe Ser Ala Gly Gly Thr Ala Ser Lys
   65                  70                  75                  80
   Gln Tyr Ala Pro Leu Phe Ala Ala Ala Gly Ala Thr Val Val Asp Asn
                   85                  90                  95
   Ser Ser Ala Trp Arg Lys Asp Asp Glu Val Pro Leu Ile Val Ser Glu
               100                 105                 110
   Val Asn Pro Ser Asp Lys Asp Ser Leu Val Lys Gly Ile Ile Ala Asn
           115                 120                 125
   Pro Asn Cys Thr Thr Met Ala Ala Met Pro Val Leu Lys Pro Leu His
       130                 135                 140
   Asp Ala Ala Gly Leu Val Lys Leu His Val Ser Ser Tyr Gln Ala Val
   145                 150                 155                 160
   Ser Gly Ser Gly Leu Ala Gly Val Glu Thr Leu Ala Lys Gln Val Ala
                   165                 170                 175
   Ala Val Gly Asp His Asn Val Glu Phe Val His Asp Gly Gln Ala Ala
               180                 185                 190
   Asp Ala Gly Asp Val Gly Pro Tyr Val Ser Pro Ile Ala Tyr Asn Val
           195                 200                 205
   Leu Pro Phe Ala Gly Asn Leu Val Asp Asp Gly Thr Phe Glu Thr Asp
       210                 215                 220
   Glu Glu Gln Lys Leu Arg Asn Glu Ser Arg Lys Ile Leu Gly Leu Pro
   225                 230                 235                 240
   Asp Leu Lys Val Ser Gly Thr Cys Val Arg Val Pro Val Phe Thr Gly
                   245                 250                 255
   His Thr Leu Thr Ile His Ala Glu Phe Asp Lys Ala Ile Thr Val Glu
               260                 265                 270
   Gln Ala Gln Glu Ile Leu Gly Ala Ala Ser Gly Val Glu Leu Val Asp
           275                 280                 285
   Val Pro Thr Pro Leu Ala Ala Ala Gly Ile Asp Glu Ser Leu Val Gly
       290                 295                 300
   Arg Ile Arg Gln Asp Ser Thr Val Asp Asp Asn Arg Gly Leu Val Leu
   305                 310                 315                 320
   Val Val Ser Gly Asp Asn Leu Arg Lys Gly Ala Ala Leu Asn Thr Ile
                   325                 330                 335
   Gln Ile Ala Glu Leu Leu Val Lys
               340
<210> 5
   <211> 747
   <212> DNA
   <213> Corynebacterium glutamicum
<220>
   <221> CDS
   <222> (1)..(747)
<400> 5
   atg gga atc aag gtt ggc gtt ctc gga gcc aaa ggc cgt gtt ggt caa       48
   Met Gly Ile Lys Val Gly Val Leu Gly Ala Lys Gly Arg Val Gly Gln
   1               5                   10                  15
   act att gtg gca gca gtc aat gag tcc gac gat ctg gag ctt gtt gca      96
   Thr Ile Val Ala Ala Val Asn Glu Ser Asp Asp Leu Glu Leu Val Ala
               20                  25                  30
   gag atc ggc gtc gac gat gat ttg agc ctt ctg gta gac aac ggc gct     144
   Glu Ile Gly Val Asp Asp Asp Leu Ser Leu Leu Val Asp Asn Gly Ala
           35                  40                  45
   gaa gtt gtc gtt gac ttc acc act cct aac gct gtg atg ggc aac ctg     192
   Glu Val Val Val Asp Phe Thr Thr Pro Asn Ala Val Met Gly Asn Leu
       50                  55                  60
   gag ttc tgc atc aac aac ggc att tct gcg gtt gtt gga acc acg ggc     240
   Glu Phe Cys Ile Asn Asn Gly Ile Ser Ala Val Val Gly Thr Thr Gly
   65                  70                  75                  80
   ttc gat aat gct cgt ttg gag cag gtt cgc gcc tgg ctt gaa gga aaa     288
   Phe Asp Asn Ala Arg Leu Glu Gln Val Arg Ala Trp Leu Glu Gly Lys
                   85                  90                  95
   gac aat gtc ggt gtt ctg atc gca cct aac ttt gct atc tct gcg gtg     336
   Asp Asn Val Gly Val Leu Ile Ala Pro Asn Phe Ala Ile Ser Ala Val
               100                 105                 110
   ttg acc atg gtc ttt tcc aag cag gct gcc cgc ttc ttc gaa tca gct     384
   Leu Thr Met Val Phe Ser Lys Gln Ala Ala Arg Phe Phe Glu Ser Ala
           115                 120                 125
   gaa gtt att gag ctg cac cac ccc aac aag ctg gat gca cct tca ggc     432
   Glu Val Ile Glu Leu His His Pro Asn Lys Leu Asp Ala Pro Ser Gly
       130                 135                 140
   acc gcg atc cac act gct cag ggc att gct gcg gca cgc aaa gaa gca     480
   Thr Ala Ile His Thr Ala Gln Gly Ile Ala Ala Ala Arg Lys Glu Ala
   145                 150                 155                 160
   ggc atg gac gca cag cca gat gcg acc gag cag gca ctt gag ggt tcc     528
   Gly Met Asp Ala Gln Pro Asp Ala Thr Glu Gln Ala Leu Glu Gly Ser
                   165                 170                 175
   cgt ggc gca agc gta gat gga atc cca gtt cac gca gtc cgc atg tcc     576
   Arg Gly Ala Ser Val Asp Gly Ile Pro Val His Ala Val Arg Met Ser
               180                 185                 190
   ggc atg gtt gct cac gag caa gtt atc ttt ggc acc cag ggt cag acc     624
   Gly Met Val Ala His Glu Gln Val Ile Phe Gly Thr Gln Gly Gln Thr
           195                 200                 205
   ttg acc atc aag cag gac tcc tat gat cgc aac tca ttt gca cca ggt     672
   Leu Thr Ile Lys Gln Asp Ser Tyr Asp Arg Asn Ser Phe Ala Pro Gly
       210                 215                 220
   gtc ttg gtg ggt gtg cgc aac att gca cag cac cca ggc cta gtc gta     720
   Val Leu Val Gly Val Arg Asn Ile Ala Gln His Pro Gly Leu Val Val
   225                 230                 235                 240
   gga ctt gag cat tac cta ggc ctg taa                                 747
   Gly Leu Glu His Tyr Leu Gly Leu
                   245
<210> 6
   <211> 248
   <212> PRT
   <213> Corynebacterium glutamicum
<400> 6
   Met Gly Ile Lys Val Gly Val Leu Gly Ala Lys Gly Arg Val Gly Gln
   1               5                   10                  15
   Thr Ile Val Ala Ala Val Asn Glu Ser Asp Asp Leu Glu Leu Val Ala
               20                  25                  30
   Glu Ile Gly Val Asp Asp Asp Leu Ser Leu Leu Val Asp Asn Gly Ala
           35                  40                  45
   Glu Val Val Val Asp Phe Thr Thr Pro Asn Ala Val Met Gly Asn Leu
       50                  55                  60
   Glu Phe Cys Ile Asn Asn Gly Ile Ser Ala Val Val Gly Thr Thr Gly
   65                  70                  75                  80
   Phe Asp Asn Ala Arg Leu Glu Gln Val Arg Ala Trp Leu Glu Gly Lys
                   85                  90                  95
   Asp Asn Val Gly Val Leu Ile Ala Pro Asn Phe Ala Ile Ser Ala Val
               100                 105                 110
   Leu Thr Met Val Phe Ser Lys Gln Ala Ala Arg Phe Phe Glu Ser Ala
           115                 120                 125
   Glu Val Ile Glu Leu His His Pro Asn Lys Leu Asp Ala Pro Ser Gly
       130                 135                 140
   Thr Ala Ile His Thr Ala Gln Gly Ile Ala Ala Ala Arg Lys Glu Ala
   145                 150                 155                 160
   Gly Met Asp Ala Gln Pro Asp Ala Thr Glu Gln Ala Leu Glu Gly Ser
                   165                 170                 175
   Arg Gly Ala Ser Val Asp Gly Ile Pro Val His Ala Val Arg Met Ser
               180                 185                 190
   Gly Met Val Ala His Glu Gln Val Ile Phe Gly Thr Gln Gly Gln Thr
           195                 200                 205
   Leu Thr Ile Lys Gln Asp Ser Tyr Asp Arg Asn Ser Phe Ala Pro Gly
       210                 215                 220
   Val Leu Val Gly Val Arg Asn Ile Ala Gln His Pro Gly Leu Val Val
   225                 230                 235                 240
   Gly Leu Glu His Tyr Leu Gly Leu
                   245
<210> 7
   <211> 1023
   <212> DNA
   <213> Corynebacterium glutamicum
<220>
   <221> CDS
   <222> (1)..(1023)
<400> 7
   atg cat ttc ggt aag ctc gac cag gac agt gcc acc aca att ttg gag      48
   Met His Phe Gly Lys Leu Asp Gln Asp Ser Ala Thr Thr Ile Leu Glu
   1               5                   10                  15
   gat tac aag aac atg acc aac atc cgc gta gct atc gta ggc tac gga      96
   Asp Tyr Lys Asn Met Thr Asn Ile Arg Val Ala Ile Val Gly Tyr Gly
               20                  25                  30
   aac ctg gga cgc agc gtc gaa aag ctt att gcc aag cag ccc gac atg      144
   Asn Leu Gly Arg Ser Val Glu Lys Leu Ile Ala Lys Gln Pro Asp Met
           35                  40                  45
   gac ctt gta gga atc ttc tcg cgc cgg gcc acc ctc gac aca aag acg      192
   Asp Leu Val Gly Ile Phe Ser Arg Arg Ala Thr Leu Asp Thr Lys Thr
       50                  55                  60
   cca gtc ttt gat gtc gcc gac gtg gac aag cac gcc gac gac gtg gac      240
   Pro Val Phe Asp Val Ala Asp Val Asp Lys His Ala Asp Asp Val Asp
   65                  70                  75                  80
   gtg ctg ttc ctg tgc atg ggc tcc gcc acc gac atc cct gag cag gca      288
   Val Leu Phe Leu Cys Met Gly Ser Ala Thr Asp Ile Pro Glu Gln Ala
                   85                  90                  95
   cca aag ttc gcg cag ttc gcc tgc acc gta gac acc tac gac aac cac      336
   Pro Lys Phe Ala Gln Phe Ala Cys Thr Val Asp Thr Tyr Asp Asn His
               100                 105                 110
   cgc gac atc cca cgc cac cgc cag gtc atg aac gaa gcc gcc acc gca      384
   Arg Asp Ile Pro Arg His Arg Gln Val Met Asn Glu Ala Ala Thr Ala
           115                 120                 125
   gcc ggc aac gtt gca ctg gtc tct acc ggc tgg gat cca gga atg ttc      432
   Ala Gly Asn Val Ala Leu Val Ser Thr Gly Trp Asp Pro Gly Met Phe
       130                 135                 140
   tcc atc aac cgc gtc tac gca gcg gca gtc tta gcc gag cac cag cag      480
   Ser Ile Asn Arg Val Tyr Ala Ala Ala Val Leu Ala Glu His Gln Gln
   145                 150                 155                 160
   cac acc ttc tgg ggc cca ggt ttg tca cag ggc cac tcc gat gct ttg      528
   His Thr Phe Trp Gly Pro Gly Leu Ser Gln Gly His Ser Asp Ala Leu
                   165                 170                 175
   cga cgc atc cct ggc gtt caa aag gcc gtc cag tac acc ctc cca tcc      576
   Arg Arg Ile Pro Gly Val Gln Lys Ala Val Gln Tyr Thr Leu Pro Ser
               180                 185                 190
   gaa gaa gcc ctg gaa aag gcc cgc cgt ggc gaa gcc ggc gac ctc acc      624
   Glu Glu Ala Leu Glu Lys Ala Arg Arg Gly Glu Ala Gly Asp Leu Thr
           195                 200                 205
   gga aag caa acc cac aag cgc caa tgc ttc gtg gtt gcc gac gcg gcc      672
   Gly Lys Gln Thr His Lys Arg Gln Cys Phe Val Val Ala Asp Ala Ala
       210                 215                 220
   gac cac gag cgc atc gaa aac gac atc cgc acc atg cct gat tac ttc      720
   Asp His Glu Arg Ile Glu Asn Asp Ile Arg Thr Met Pro Asp Tyr Phe
   225                 230                 235                 240
   gtt ggc tac gaa gtc gaa gtc aac ttc atc gac gaa gca acc ttg gac      768
   Val Gly Tyr Glu Val Glu Val Asn Phe Ile Asp Glu Ala Thr Leu Asp
                   245                 250                 255
   gcc gag cac acc ggc atg cca cac ggc gga cac gtg atc acc acc ggc      816
   Ala Glu His Thr Gly Met Pro His Gly Gly His Val Ile Thr Thr Gly
               260                 265                 270
   gac acc ggt ggc ttc aac cac acc gtg gaa tac atc ctg aag ctg gac      864
   Asp Thr Gly Gly Phe Asn His Thr Val Glu Tyr Ile Leu Lys Leu Asp
           275                 280                 285
   cga aac cca gat ttc acc gct tct tca cag atc gct ttc ggc cgc gca      912
   Arg Asn Pro Asp Phe Thr Ala Ser Ser Gln Ile Ala Phe Gly Arg Ala
       290                 295                 300
   gct cac cgc atg aag cag cag ggc caa agc ggt gct ttc acc gtc ctc      960
   Ala His Arg Met Lys Gln Gln Gly Gln Ser Gly Ala Phe Thr Val Leu
   305                 310                 315                 320
   gaa gtt gct cca tac ttg ctc tcc ccg gag aac ttg gat gat ctg atc     1008
   Glu Val Ala Pro Tyr Leu Leu Ser Pro Glu Asn Leu Asp Asp Leu Ile
                   325                 330                 335
   gca cgc gac gtc taa                                                 1023
   Ala Arg Asp Val
               340
<210> 8
   <211> 340
   <212> PRT
   <213> Corynebacterium glutamicum
<400> 8
   Met His Phe Gly Lys Leu Asp Gln Asp Ser Ala Thr Thr Ile Leu Glu
   1               5                   10                  15
   Asp Tyr Lys Asn Met Thr Asn Ile Arg Val Ala Ile Val Gly Tyr Gly
               20                  25                  30
   Asn Leu Gly Arg Ser Val Glu Lys Leu Ile Ala Lys Gln Pro Asp Met
           35                  40                  45
   Asp Leu Val Gly Ile Phe Ser Arg Arg Ala Thr Leu Asp Thr Lys Thr
       50                  55                  60
   Pro Val Phe Asp Val Ala Asp Val Asp Lys His Ala Asp Asp Val Asp
   65                  70                  75                  80
   Val Leu Phe Leu Cys Met Gly Ser Ala Thr Asp Ile Pro Glu Gln Ala
                   85                  90                  95
   Pro Lys Phe Ala Gln Phe Ala Cys Thr Val Asp Thr Tyr Asp Asn His
               100                 105                 110
   Arg Asp Ile Pro Arg His Arg Gln Val Met Asn Glu Ala Ala Thr Ala
           115                 120                 125
   Ala Gly Asn Val Ala Leu Val Ser Thr Gly Trp Asp Pro Gly Met Phe
       130                 135                 140
   Ser Ile Asn Arg Val Tyr Ala Ala Ala Val Leu Ala Glu His Gln Gln
   145                 150                 155                 160
   His Thr Phe Trp Gly Pro Gly Leu Ser Gln Gly His Ser Asp Ala Leu
                   165                 170                 175
   Arg Arg Ile Pro Gly Val Gln Lys Ala Val Gln Tyr Thr Leu Pro Ser
               180                 185                 190
   Glu Glu Ala Leu Glu Lys Ala Arg Arg Gly Glu Ala Gly Asp Leu Thr
           195                 200                 205
   Gly Lys Gln Thr His Lys Arg Gln Cys Phe Val Val Ala Asp Ala Ala
       210                 215                 220
   Asp His Glu Arg Ile Glu Asn Asp Ile Arg Thr Met Pro Asp Tyr Phe
   225                 230                 235                 240
   Val Gly Tyr Glu Val Glu Val Asn Phe Ile Asp Glu Ala Thr Leu Asp
                   245                 250                 255
   Ala Glu His Thr Gly Met Pro His Gly Gly His Val Ile Thr Thr Gly
               260                 265                 270
   Asp Thr Gly Gly Phe Asn His Thr Val Glu Tyr Ile Leu Lys Leu Asp
           275                 280                 285
   Arg Asn Pro Asp Phe Thr Ala Ser Ser Gln Ile Ala Phe Gly Arg Ala
       290                 295                 300
   Ala His Arg Met Lys Gln Gln Gly Gln Ser Gly Ala Phe Thr Val Leu
   305                 310                 315                 320
   Glu Val Ala Pro Tyr Leu Leu Ser Pro Glu Asn Leu Asp Asp Leu Ile
                   325                 330                 335
   Ala Arg Asp Val
               340
<210> 9
   <211> 753
   <212> DNA
   <213> Corynebacterium glutamicum
<220>
   <221> CDS
   <222> (1)..(753)
<400> 9
   gtg gcc gaa caa gtt aaa ttg agc gtg gag ttg ata gcg tgc agt tct      48
   Met Ala Glu Gln Val Lys Leu Ser Val Glu Leu Ile Ala Cys Ser Ser
   1               5                   10                  15
   ttt act cca ccc gct gat gtt gag tgg tca act gat gtt gag ggc gcg      96
   Phe Thr Pro Pro Ala Asp Val Glu Trp Ser Thr Asp Val Glu Gly Ala
               20                  25                  30
   gaa gca ctc gtc gag ttt gcg ggt cgt gcc tgc tac gaa act ttt gat     144
   Glu Ala Leu Val Glu Phe Ala Gly Arg Ala Cys Tyr Glu Thr Phe Asp
           35                  40                  45
   aag ccg aac cct cga act gct tcc aat gct gcg tat ctg cgc cac atc     192
   Lys Pro Asn Pro Arg Thr Ala Ser Asn Ala Ala Tyr Leu Arg His Ile
       50                  55                  60
   atg gaa gtg ggg cac act gct ttg ctt gag cat gcc aat gcc acg atg      240
   Met Glu Val Gly His Thr Ala Leu Leu Glu His Ala Asn Ala Thr Met
   65                  70                  75                  80
   tat atc cga ggc att tct cgg tcc gcg acc cat gaa ttg gtc cga cac      288
   Tyr Ile Arg Gly Ile Ser Arg Ser Ala Thr His Glu Leu Val Arg His
                   85                  90                  95
   cgc cat ttt tcc ttc tct caa ctg tct cag cgt ttc gtg cac agc gga      336
   Arg His Phe Ser Phe Ser Gln Leu Ser Gln Arg Phe Val His Ser Gly
               100                 105                 110
   gaa tcg gaa gta gtg gtg ccc act ctc atc gat gaa gat ccg cag ttg      384
   Glu Ser Glu Val Val Val Pro Thr Leu Ile Asp Glu Asp Pro Gln Leu
           115                 120                 125
   cgt gaa ctt ttc atg cac gcc atg gat gag tct cgg ttc gct ttc aat      432
   Arg Glu Leu Phe Met His Ala Met Asp Glu Ser Arg Phe Ala Phe Asn
       130                 135                 140
   gag ctg ctt aat gcg ctg gaa gaa aaa ctt ggc gat gaa ccg aat gca      480
   Glu Leu Leu Asn Ala Leu Glu Glu Lys Leu Gly Asp Glu Pro Asn Ala
   145                 150                 155                 160
   ctt tta agg aaa aag cag gct cgt caa gca gct cgc gct gtg ctg ccc      528
   Leu Leu Arg Lys Lys Gln Ala Arg Gln Ala Ala Arg Ala Val Leu Pro
                   165                 170                 175
   aac gct aca gag tcc aga atc gtg gtg tct gga aac ttc cgc acc tgg      576
   Asn Ala Thr Glu Ser Arg Ile Val Val Ser Gly Asn Phe Arg Thr Trp
               180                 185                 190
   agg cat ttc att ggc atg cga gcc agt gaa cat gca gac gtc gaa atc      624
   Arg His Phe Ile Gly Met Arg Ala Ser Glu His Ala Asp Val Glu Ile
           195                 200                 205
   cgc gaa gta gcg gta gga tgt tta aga aag ctg cag gta gca gcg cca      672
   Arg Glu Val Ala Val Gly Cys Leu Arg Lys Leu Gln Val Ala Ala Pro
       210                 215                 220
   act gtt ttc ggt gat ttt gag att gaa act ttg gca gac gga tcg caa      720
   Thr Val Phe Gly Asp Phe Glu Ile Glu Thr Leu Ala Asp Gly Ser Gln
   225                 230                 235                 240
   atg gca aca agc ccg tat gtc atg gac ttt taa                          753
   Met Ala Thr Ser Pro Tyr Val Met Asp Phe
                   245                 250
<210> 10
   <211> 250
   <212> PRT
   <213> Corynebacterium glutamicum
<400> 10
   Met Ala Glu Gln Val Lys Leu Ser Val Glu Leu Ile Ala Cys Ser Ser
   1               5                   10                  15
   Phe Thr Pro Pro Ala Asp Val Glu Trp Ser Thr Asp Val Glu Gly Ala
               20                  25                  30
   Glu Ala Leu Val Glu Phe Ala Gly Arg Ala Cys Tyr Glu Thr Phe Asp
           35                  40                  45
   Lys Pro Asn Pro Arg Thr Ala Ser Asn Ala Ala Tyr Leu Arg His Ile
       50                  55                  60
   Met Glu Val Gly His Thr Ala Leu Leu Glu His Ala Asn Ala Thr Met
   65                  70                  75                  80
   Tyr Ile Arg Gly Ile Ser Arg Ser Ala Thr His Glu Leu Val Arg His
                   85                  90                  95
   Arg His Phe Ser Phe Ser Gln Leu Ser Gln Arg Phe Val His Ser Gly
               100                 105                 110
   Glu Ser Glu Val Val Val Pro Thr Leu Ile Asp Glu Asp Pro Gln Leu
           115                 120                 125
   Arg Glu Leu Phe Met His Ala Met Asp Glu Ser Arg Phe Ala Phe Asn
       130                 135                 140
   Glu Leu Leu Asn Ala Leu Glu Glu Lys Leu Gly Asp Glu Pro Asn Ala
   145                 150                 155                 160
   Leu Leu Arg Lys Lys Gln Ala Arg Gln Ala Ala Arg Ala Val Leu Pro
                   165                 170                 175
   Asn Ala Thr Glu Ser Arg Ile Val Val Ser Gly Asn Phe Arg Thr Trp
               180                 185                 190
   Arg His Phe Ile Gly Met Arg Ala Ser Glu His Ala Asp Val Glu Ile
           195                 200                 205
   Arg Glu Val Ala Val Gly Cys Leu Arg Lys Leu Gln Val Ala Ala Pro
       210                 215                 220
   Thr Val Phe Gly Asp Phe Glu Ile Glu Thr Leu Ala Asp Gly Ser Gln
   225                 230                 235                 240
   Met Ala Thr Ser Pro Tyr Val Met Asp Phe
                   245                 250
<210> 11
   <211> 1338
   <212> DNA
   <213> Corynebacterium glutamicum
<220>
   <221> CDS
   <222> (1)..(1338)
<400> 11
   atg gct aca gtt gaa aat ttc aat gaa ctt ccc gca cac gta tgg cca       48
   Met Ala Thr Val Glu Asn Phe Asn Glu Leu Pro Ala His Val Trp Pro
   1               5                   10                  15
   cgc aat gcc gtg cgc caa gaa gac ggc gtt gtc acc gtc gct ggt gtg       96
   Arg Asn Ala Val Arg Gln Glu Asp Gly Val Val Thr Val Ala Gly Val
               20                  25                  30
   cct ctg cct gac ctc gct gaa gaa tac gga acc cca ctg ttc gta gtc      144
   Pro Leu Pro Asp Leu Ala Glu Glu Tyr Gly Thr Pro Leu Phe Val Val
           35                  40                  45
   gac gag gac gat ttc cgt tcc cgc tgt cgc gac atg gct acc gca ttc      192
   Asp Glu Asp Asp Phe Arg Ser Arg Cys Arg Asp Met Ala Thr Ala Phe
       50                  55                  60
   ggt gga cca ggc aat gtg cac tac gca tct aaa gcg ttc ctg acc aag      240
   Gly Gly Pro Gly Asn Val His Tyr Ala Ser Lys Ala Phe Leu Thr Lys
   65                  70                  75                  80
   acc att gca cgt tgg gtt gat gaa gag ggg ctg gca ctg gac att gca      288
   Thr Ile Ala Arg Trp Val Asp Glu Glu Gly Leu Ala Leu Asp Ile Ala
                   85                  90                  95
   tcc atc aac gaa ctg ggc att gcc ctg gcc gct ggt ttc ccc gcc agc      336
   Ser Ile Asn Glu Leu Gly Ile Ala Leu Ala Ala Gly Phe Pro Ala Ser
               100                 105                 110
   cgt atc acc gcg cac ggc aac aac aaa ggc gta gag ttc ctg cgc gcg      384
   Arg Ile Thr Ala His Gly Asn Asn Lys Gly Val Glu Phe Leu Arg Ala
           115                 120                 125
   ttg gtt caa aac ggt gtg gga cac gtg gtg ctg gac tcc gca cag gaa      432
   Leu Val Gln Asn Gly Val Gly His Val Val Leu Asp Ser Ala Gln Glu
       130                 135                 140
   cta gaa ctg ttg gat tac gtt gcc gct ggt gaa ggc aag att cag gac      480
   Leu Glu Leu Leu Asp Tyr Val Ala Ala Gly Glu Gly Lys Ile Gln Asp
   145                 150                 155                 160
   gtg ttg atc cgc gta aag cca ggc atc gaa gca cac acc cac gag ttc      528
   Val Leu Ile Arg Val Lys Pro Gly Ile Glu Ala His Thr His Glu Phe
                   165                 170                 175
   atc gcc act agc cac gaa gac cag aag ttc gga ttc tcc ctg gca tcc      576
   Ile Ala Thr Ser His Glu Asp Gln Lys Phe Gly Phe Ser Leu Ala Ser
               180                 185                 190
   ggt tcc gca ttc gaa gca gca aaa gcc gcc aac aac gca gaa aac ctg       624
   Gly Ser Ala Phe Glu Ala Ala Lys Ala Ala Asn Asn Ala Glu Asn Leu
           195                 200                 205
   aac ctg gtt ggc ctg cac tgc cac gtt ggt tcc cag gtg ttc gac gcc       672
   Asn Leu Val Gly Leu His Cys His Val Gly Ser Gln Val Phe Asp Ala
       210                 215                 220
   gaa ggc ttc aag ctg gca gca gaa cgc gtg ttg ggc ctg tac tca cag       720
   Glu Gly Phe Lys Leu Ala Ala Glu Arg Val Leu Gly Leu Tyr Ser Gln
   225                 230                 235                 240
   atc cac agc gaa ctg ggc gtt gcc ctt cct gaa ctg gat ctc ggt ggc       768
   Ile His Ser Glu Leu Gly Val Ala Leu Pro Glu Leu Asp Leu Gly Gly
                   245                 250                 255
   gga tac ggc att gcc tat acc gca gct gaa gaa cca ctc aac gtc gca       816
   Gly Tyr Gly Ile Ala Tyr Thr Ala Ala Glu Glu Pro Leu Asn Val Ala
               260                 265                 270
   gaa gtt gcc tcc gac ctg ctc acc gca gtc gga aaa atg gca gcg gaa       864
   Glu Val Ala Ser Asp Leu Leu Thr Ala Val Gly Lys Met Ala Ala Glu
           275                 280                 285
   cta ggc atc gac gca cca acc gtg ctt gtt gag ccc ggc cgc gct atc       912
   Leu Gly Ile Asp Ala Pro Thr Val Leu Val Glu Pro Gly Arg Ala Ile
       290                 295                 300
   gca ggc ccc tcc acc gtg acc atc tac gaa gtc ggc acc acc aaa gac       960
   Ala Gly Pro Ser Thr Val Thr Ile Tyr Glu Val Gly Thr Thr Lys Asp
   305                 310                 315                 320
   gtc cac gta gac gac gac aaa acc cgc cgt tac atc gcc gtg gac gga      1008
   Val His Val Asp Asp Asp Lys Thr Arg Arg Tyr Ile Ala Val Asp Gly
                   325                 330                 335
   ggc atg tcc gac aac atc cgc cca gca ctc tac ggc tcc gaa tac gac      1056
   Gly Met Ser Asp Asn Ile Arg Pro Ala Leu Tyr Gly Ser Glu Tyr Asp
               340                 345                 350
   gcc cgc gta gta tcc cgc ttc gcc gaa gga gac cca gta agc acc cgc      1104
   Ala Arg Val Val Ser Arg Phe Ala Glu Gly Asp Pro Val Ser Thr Arg
           355                 360                 365
   atc gtg ggc tcc cac tgc gaa tcc ggc gat atc ctg atc aac gat gaa      1152
   Ile Val Gly Ser His Cys Glu Ser Gly Asp Ile Leu Ile Asn Asp Glu
       370                 375                 380
   atc tac cca tct gac atc acc agc ggc gac ttc ctt gca ctc gca gcc      1200
   Ile Tyr Pro Ser Asp Ile Thr Ser Gly Asp Phe Leu Ala Leu Ala Ala
   385                 390                 395                 400
   acc ggc gca tac tgc tac gcc atg agc tcc cgc tac aac gcc ttc aca      1248
   Thr Gly Ala Tyr Cys Tyr Ala Met Ser Ser Arg Tyr Asn Ala Phe Thr
                   405                 410                 415
   cgg ccc gcc gtc gtg tcc gtc cgc gct ggc agc tcc cgc ctc atg ctg      1296
   Arg Pro Ala Val Val Ser Val Arg Ala Gly Ser Ser Arg Leu Met Leu
               420                 425                 430
   cgc cgc gaa acg ctc gac gac atc ctc tca cta gag gca taa              1338
   Arg Arg Glu Thr Leu Asp Asp Ile Leu Ser Leu Glu Ala
           435                 440                 445
<210> 12
   <211> 445
   <212> PRT
   <213> Corynebacterium glutamicum
<400> 12
   Met Ala Thr Val Glu Asn Phe Asn Glu Leu Pro Ala His Val Trp Pro
   1               5                   10                  15
   Arg Asn Ala Val Arg Gln Glu Asp Gly Val Val Thr Val Ala Gly Val
               20                  25                  30
   Pro Leu Pro Asp Leu Ala Glu Glu Tyr Gly Thr Pro Leu Phe Val Val
           35                  40                  45
   Asp Glu Asp Asp Phe Arg Ser Arg Cys Arg Asp Met Ala Thr Ala Phe
       50                  55                  60
   Gly Gly Pro Gly Asn Val His Tyr Ala Ser Lys Ala Phe Leu Thr Lys
   65                  70                  75                  80
   Thr Ile Ala Arg Trp Val Asp Glu Glu Gly Leu Ala Leu Asp Ile Ala
                   85                  90                  95
   Ser Ile Asn Glu Leu Gly Ile Ala Leu Ala Ala Gly Phe Pro Ala Ser
               100                 105                 110
   Arg Ile Thr Ala His Gly Asn Asn Lys Gly Val Glu Phe Leu Arg Ala
           115                 120                 125
   Leu Val Gln Asn Gly Val Gly His Val Val Leu Asp Ser Ala Gln Glu
       130                 135                 140
   Leu Glu Leu Leu Asp Tyr Val Ala Ala Gly Glu Gly Lys Ile Gln Asp
   145                 150                 155                 160
   Val Leu Ile Arg Val Lys Pro Gly Ile Glu Ala His Thr His Glu Phe
                   165                 170                 175
   Ile Ala Thr Ser His Glu Asp Gln Lys Phe Gly Phe Ser Leu Ala Ser
               180                 185                 190
   Gly Ser Ala Phe Glu Ala Ala Lys Ala Ala Asn Asn Ala Glu Asn Leu
           195                 200                 205
   Asn Leu Val Gly Leu His Cys His Val Gly Ser Gln Val Phe Asp Ala
       210                 215                 220
   Glu Gly Phe Lys Leu Ala Ala Glu Arg Val Leu Gly Leu Tyr Ser Gln
   225                 230                 235                 240
   Ile His Ser Glu Leu Gly Val Ala Leu Pro Glu Leu Asp Leu Gly Gly
                   245                 250                 255
   Gly Tyr Gly Ile Ala Tyr Thr Ala Ala Glu Glu Pro Leu Asn Val Ala
               260                 265                 270
   Glu Val Ala Ser Asp Leu Leu Thr Ala Val Gly Lys Met Ala Ala Glu
           275                 280                 285
   Leu Gly Ile Asp Ala Pro Thr Val Leu Val Glu Pro Gly Arg Ala Ile
       290                 295                 300
   Ala Gly Pro Ser Thr Val Thr Ile Tyr Glu Val Gly Thr Thr Lys Asp
   305                 310                 315                 320
   Val His Val Asp Asp Asp Lys Thr Arg Arg Tyr Ile Ala Val Asp Gly
                   325                 330                 335
   Gly Met Ser Asp Asn Ile Arg Pro Ala Leu Tyr Gly Ser Glu Tyr Asp
               340                 345                 350
   Ala Arg Val Val Ser Arg Phe Ala Glu Gly Asp Pro Val Ser Thr Arg
           355                 360                 365
   Ile Val Gly Ser His Cys Glu Ser Gly Asp Ile Leu Ile Asn Asp Glu
       370                 375                 380
   Ile Tyr Pro Ser Asp Ile Thr Ser Gly Asp Phe Leu Ala Leu Ala Ala
   385                 390                 395                 400
   Thr Gly Ala Tyr Cys Tyr Ala Met Ser Ser Arg Tyr Asn Ala Phe Thr
                   405                 410                 415
   Arg Pro Ala Val Val Ser Val Arg Ala Gly Ser Ser Arg Leu Met Leu
               420                 425                 430
   Arg Arg Glu Thr Leu Asp Asp Ile Leu Ser Leu Glu Ala
           435                 440                 445
<210> 13
   <211> 365
   <212> DNA
   <213> Corynebacterium glutamicum
<220>
   <221> CDS
   <222> (1)..(365)
<400> 13
   gtg gcc gaa caa gtt aaa ttg agc gtg gag ttg ata gcg tgc agt tct        48
   Met Ala Glu Gln Val Lys Leu Ser Val Glu Leu Ile Ala Cys Ser Ser
   1               5                   10                  15
   ttt act cca ccc gct gat gtt gag tgg tca act gat gtt gag ggc gcg        96
   Phe Thr Pro Pro Ala Asp Val Glu Trp Ser Thr Asp Val Glu Gly Ala
               20                  25                  30
   gaa gca ctc gtc gag ttt gcg ggt cgt gcc tgc tac gaa act ttt gat       144
   Glu Ala Leu Val Glu Phe Ala Gly Arg Ala Cys Tyr Glu Thr Phe Asp
           35                  40                  45
   aag ccg aac cct cga act gct tcc aat gct gcg tat ctg cgc cac atc       192
   Lys Pro Asn Pro Arg Thr Ala Ser Asn Ala Ala Tyr Leu Arg His Ile
       50                  55                  60
   atg gaa gtg ggg cac act gct ttg ctt gag cat gcc aat gcc acg atg       240
   Met Glu Val Gly His Thr Ala Leu Leu Glu His Ala Asn Ala Thr Met
   65                  70                  75                  80
   tat atc cga ggc att tct cgg tcc gcg acc cat gaa ttg gtc cga cac       288
   Tyr Ile Arg Gly Ile Ser Arg Ser Ala Thr His Glu Leu Val Arg His
                   85                  90                  95
   cgc cat ttt tcc ttc tct caa ctg tct cag cgt ttc gtg cac agc gga       336
   Arg His Phe Ser Phe Ser Gln Leu Ser Gln Arg Phe Val His Ser Gly
               100                 105                 110
   gaa tcg gaa gta gtg gtg ccc act ctc at                                365
   Glu Ser Glu Val Val Val Pro Thr Leu Ile
           115                 120
<210> 14
   <211> 122
   <212> PRT
   <213> Corynebacterium glutamicum
<400> 14
   Met Ala Glu Gln Val Lys Leu Ser Val Glu Leu Ile Ala Cys Ser Ser
   1               5                   10                  15
   Phe Thr Pro Pro Ala Asp Val Glu Trp Ser Thr Asp Val Glu Gly Ala
               20                  25                  30
   Glu Ala Leu Val Glu Phe Ala Gly Arg Ala Cys Tyr Glu Thr Phe Asp
           35                  40                  45
   Lys Pro Asn Pro Arg Thr Ala Ser Asn Ala Ala Tyr Leu Arg His Ile
       50                  55                  60
   Met Glu Val Gly His Thr Ala Leu Leu Glu His Ala Asn Ala Thr Met
   65                  70                  75                  80
   Tyr Ile Arg Gly Ile Ser Arg Ser Ala Thr His Glu Leu Val Arg His
                   85                  90                  95
   Arg His Phe Ser Phe Ser Gln Leu Ser Gln Arg Phe Val His Ser Gly
               100                 105                 110
   Glu Ser Glu Val Val Val Pro Thr Leu Ile
           115                 120
<210> 15
   <211> 551
   <212> DNA
   <213> Corynebacterium glutamicum
<400> 15
   aaccggtgtg gagccgacca ttccgcgagg ctgcactgca acgaggtcgt agttttggta      60
   catggcttct ggccagttca tggattggct gccgaagaag ctataggcat cgccaccagg     120
   gccaccggag ttaccgaaga tggtgccgtg cttttcgcct tgggcaggga ccttgacaaa     180
   gcccacgctg atatcgccaa gtgagggatc agaatagtgc atgggcacgt cgatgctgcc     240
   acattgagcg gaggcaatat ctacctgagg tgggcattct tcccagcgga tgttttcttg     300
   cgctgctgca gtgggcattg ataccaaaaa ggggctaagc gcagtcgagg cggcaagaac     360
   tgctactacc ttttttattg tcgaacgggg cattacggct ccaaggacgt ttgttttctg     420
   ggtcagttac cccaaaaagc atatacagag accaatgatt tttcattaaa aaggcaggga     480
   tttgttataa gtatgggtcg tattctgtgc gacgggtgta cctcggctag aatttctccc     540
   catgacacca g                                                          551
<210> 16
   <211> 421
   <212> PRT
   <213> Artificial Sequence
<220>
   <223> Consensus Sequence of Protein Sequence Alignment
<220>
   <221> MISC_FEATURE
   <222> (40)..(40)
   <223> May be either Cys or Val
<220>
   <221> MISC_FEATURE
   <222> (317)..(317)
   <223> May be either Ser or Ala
<220>
   <221> MISC_FEATURE
   <222> (345)..(345)
   <223> May be either Gly or Asp
<220>
   <221> MISC_FEATURE
   <222> (380)..(380)
   <223> May be either Thr or Ile
<400> 16
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
   20 25 30
   Gly Asn Asp Val Val Val Val Xaa Ser Ala Met Gly Asp Thr Thr Asp
   35 40 45
   Glu Leu Leu Glu Leu Ala Ala Ala Val Asn Pro Val Pro Pro Ala Arg
   50 55 60
   Glu Met Asp Met Leu Leu Thr Ala Gly Glu Arg Ile Ser Asn Ala Leu
   65 70 75 80
   Val Ala Met Ala Ile Glu Ser Leu Gly Ala Glu Ala Gln Ser Phe Thr
   85 90 95
   Gly Ser Gln Ala Gly Val Leu Thr Thr Glu Arg His Gly Asn Ala Arg
   100 105 110
   Ile Val Asp Val Thr Pro Gly Arg Val Arg Glu Ala Leu Asp Glu Gly
   115 120 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Xaa Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Xaa Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Xaa Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 17
   <211> 28
   <212> DNA
   <213> Artificial
<220>
   <223> Primer
<400> 17
   gggtacctcg cgaagtagca cctgtcac 28
<210> 18
   <211> 26
   <212> DNA
   <213> Artificial
<220>
   <223> Primer
<400> 18
   gcggatcccc catcgcccct caaaga 26
<210> 19
   <211> 21
   <212> DNA
   <213> Artificial
<220>
   <223> Primer
<400> 19
   aacgggcggt gaagggcaac t 21
<210> 20
   <211> 21
   <212> DNA
   <213> Artificial
<220>
   <223> Primer
<400> 20
   tgaaagacag gggtatccag a 21
<210> 21
   <211> 24
   <212> DNA
   <213> Artificial
<220>
   <223> Primer
<400> 21
   ccatggtacc aagtgcgtgg cgag 24
<210> 22
   <211> 25
   <212> DNA
   <213> Artificial
<220>
   <223> Primer
<400> 22
   ccatggtacc acactgtttc cttgc 25
<210> 23
   <211> 25
   <212> DNA
   <213> Artificial
<220>
   <223> Primer
<400> 23
   ccggagaaga tgtaacaatg gctac 25
<210> 24
   <211> 25
   <212> DNA
   <213> Artificial
<220>
   <223> Primer
<400> 24
   cctcgactgc agacccctag acacc 25
<210> 25
   <211> 30
   <212> DNA
   <213> Artificial
<220>
   <223> Primer
<400> 25
   gctcatagag ttcaaggtta ccttcttccc 30